# EUROPEAN PATENT SPECIFICATION

(11) **EP 2 619 330 B1**
(45) Date of publication and mention of the grant of the patent: **09.05.2018**
(21) Application number: 11827554.4
(22) Date of filing: 22.09.2011
(51) Int. Cl.: C12Q 1/68, C12Q 1/37, C12N 9/48, G01N 33/50

(54) **METHOD FOR IDENTIFYING COMPOUNDS TO INCREASE THE EXPRESSION OR ACTIVITY OF ASPARTYL AMINOPEPTIDASE**
VERFAHREN ZUR IDENTIFIZIERUNG VON VERBINDUNGEN ZUR ERHÖHUNG DER EXPRESSION ODER AKTIVITÄT VON ASPARTYL-AMINOPEPTIDASE
PROCÉDÉS POUR IDENTIFIER DES COMPOSÉS PERMETTANT D'AUGMENTER L'EXPRESSION OU L'ACTIVITÉ DE L'ASPARTYL AMINOPEPTIDASE

(30) Priority: 22.09.2010 US 385322 P
(43) Date of publication of application: 31.07.2013
(73) Proprietor: Howard University, Washington, DC 20059 (US)
(72) Inventor: CHEN, Yuanxiu, Potomac Maryland 20854 (US)
(74) Representative: Pronovem
(86) International application number: PCT/US2011/052799
(87) International publication number: WO 2012/040491

(56) References cited:
- WO-A1-2005/103290
- WO-A1-2005/103290
- CAI WENDY W ET AL: "Aspartyl Aminopeptidase, Encoded by a Evolutionarily Conserved Syntenic Gene, Is Colocalized with Its Clustered Homologs in Secretory Granules of Pancreatic Glucagon Cells", DIABETES, vol. 59, no. Suppl. 1, June 2010 (2010-06) , page A447, XP009175950, & 70TH ANNUAL MEETING OF THE AMERICAN-DIABETES-ASSOCIATION; ORLANDO, FL, USA; JUNE 25 -29, 2010 ISSN: 0012-1797
- WILK E A: "Purification, characterization and cloning of a cytosolic aspartyl aminopeptidase", JOURNAL OF BIOLOGICAL CHEMISTRY.(MICROFILMS), AMERICAN SOCIETY OF BIOLOGICAL CHEMISTS, BALTIMORE, MD, US, vol. 273, no. 26, 26 June 1998 (1998-06-26), pages 15961-15970, XP002208002, DOI: 10.1074/JBC.273.26.15961
- WILK ET AL.: 'Purification, characterization, and cloning of a cytosolic aspartyl aminopeptidase.' J BIOL CHEM. vol. 273, no. 26, 26 June 1998, pages 15961 - 70, XP002208002
- CAI ET AL.: 'Aspartyl aminopeptidase, encoded by an evolutionarily conserved syntenic gene, is colocalized with its cluster in secretory granules of pancreatic islet cells.' BIOSCI BIOTECHNOL BIOCHEM. vol. 74, no. 10, 07 October 2010, pages 2050 - 2055, XP055107696

### SEQUENCE LISTING

<110> Chen, Yuanxiu
<120> Methods for Identifying Compounds Effective to Increase the Expression or Activity of Aspartyl Aminopeptidase in Mammalian Pancreatic Islet Cells, and Methods for Decreasing Angiotensin II Levels in Mammalian Pancreatic Islet Cells
<130> 8823-98230
<160> 13
<170> PatentIn version 3.5
<210> 1
   <211> 979
   <212> PRT
   <213> Homo sapiens
<400> 1
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
               180                 185                 190
   Gln Pro Pro His Pro Ser Leu Ser Tyr Glu Pro Ala Leu Leu Gln Pro
           195                 200                 205
   Tyr Leu Phe His Gln Phe Gly Ser Arg Asp Gly Ser Arg Val Ser Glu
       210                 215                 220
   Gly Ser Pro Gly Met Val Ser Val Gly Pro Leu Pro Lys Ala Glu Ala
   225                 230                 235                 240
   Pro Ala Leu Phe Ser Arg Thr Ala Ser Lys Gly Ile Phe Gly Asp His
                   245                 250                 255
   Pro Gly His Ser Tyr Gly Asp Leu Pro Gly Pro Ser Pro Ala Gln Leu
               260                 265                 270
   Phe Gln Asp Ser Gly Leu Leu Tyr Leu Ala Gln Glu Leu Pro Ala Pro
           275                 280                 285
   Ser Arg Ala Arg Val Pro Arg Leu Pro Glu Gln Gly Ser Ser Ser Arg
       290                 295                 300
   Ala Glu Asp Ser Pro Glu Gly Tyr Glu Lys Glu Gly Leu Gly Asp Arg
   305                 310                 315                 320
   Gly Glu Lys Pro Ala Ser Pro Ala Val Gln Pro Asp Ala Ala Leu Gln
                   325                 330                 335
   Arg Leu Ala Ala Val Leu Ala Gly Tyr Gly Val Glu Leu Arg Gln Leu
               340                 345                 350
   Thr Pro Glu Gln Leu Ser Thr Leu Leu Thr Leu Leu Gln Leu Leu Pro
           355                 360                 365
   Lys Gly Ala Gly Arg Asn Pro Gly Gly Val Val Asn Val Gly Ala Asp
       370                 375                 380
   Ile Lys Lys Thr Met Glu Gly Pro Val Glu Gly Arg Asp Thr Ala Glu
   385                 390                 395                 400
   Leu Pro Ala Arg Thr Ser Pro Met Pro Gly His Pro Thr Ala Ser Pro
                   405                 410                 415
   Thr Ser Ser Glu Val Gln Gln Val Pro Ser Pro Val Ser Ser Glu Pro
   420 425 430
   Pro Lys Ala Ala Arg Pro Pro Val Thr Pro Val Leu Leu Glu Lys Lys
   435 440 445
   Ser Pro Leu Gly Gln Ser Gln Pro Thr Val Ala Gly Gln Pro Ser Ala
   450 455 460
   Arg Pro Ala Ala Glu Glu Tyr Gly Tyr Ile Val Thr Asp Gln Lys Pro
   465 470 475 480
   Leu Ser Leu Ala Ala Gly Val Lys Leu Leu Glu Ile Leu Ala Glu His
   485 490 495
   Val His Met Ser Ser Gly Ser Phe Ile Asn Ile Ser Val Val Gly Pro
   500 505 510
   Ala Leu Thr Phe Arg Ile Arg His Asn Glu Gln Asn Leu Ser Leu Ala
   515 520 525
   Asp Val Thr Gln Gln Ala Gly Leu Val Lys Ser Glu Leu Glu Ala Gln
   530 535 540
   Thr Gly Leu Gln Ile Leu Gln Thr Gly Val Gly Gln Arg Glu Glu Ala
   545 550 555 560
   Ala Ala Val Leu Pro Gln Thr Ala His Ser Thr Ser Pro Met Arg Ser
   565 570 575
   Val Leu Leu Thr Leu Val Ala Leu Ala Gly Val Ala Gly Leu Leu Val
   580 585 590
   Ala Leu Ala Val Ala Leu Cys Val Arg Gln His Ala Arg Gln Gln Asp
   595 600 605
   Lys Glu Arg Leu Ala Ala Leu Gly Pro Glu Gly Ala His Gly Asp Thr
   610 615 620
   Thr Phe Glu Tyr Gln Asp Leu Cys Arg Gln His Met Ala Thr Lys Ser
   625 630 635 640
   Leu Phe Asn Arg Ala Glu Gly Pro Pro Glu Pro Ser Arg Val Ser Ser
   645 650 655
   Val Ser Ser Gln Phe Ser Asp Ala Ala Gln Ala Ser Pro Ser Ser His
   660 665 670
   Ser Ser Thr Pro Ser Trp Cys Glu Glu Pro Ala Gln Ala Asn Met Asp
   675 680 685
   Ile Ser Thr Gly His Met Ile Leu Ala Tyr Met Glu Asp His Leu Arg
   690 695 700
   Asn Arg Asp Arg Leu Ala Lys Glu Trp Gln Ala Leu Cys Ala Tyr Gln
   705 710 715 720
   Ala Glu Pro Asn Thr Cys Ala Thr Ala Gln Gly Glu Gly Asn Ile Lys
   725 730 735
   Lys Asn Arg His Pro Asp Phe Leu Pro Tyr Asp His Ala Arg Ile Lys
   740 745 750
   Leu Lys Val Glu Ser Ser Pro Ser Arg Ser Asp Tyr Ile Asn Ala Ser
   755 760 765
   Pro Ile Ile Glu His Asp Pro Arg Met Pro Ala Tyr Ile Ala Thr Gln
   770 775 780
   Gly Pro Leu Ser His Thr Ile Ala Asp Phe Trp Gln Met Val Trp Glu
   785 790 795 800
   Ser Gly Cys Thr Val Ile Val Met Leu Thr Pro Leu Val Glu Asp Gly
   805 810 815
   Val Lys Gln Cys Asp Arg Tyr Trp Pro Asp Glu Gly Ala Ser Leu Tyr
   820 825 830
   His Val Tyr Glu Val Asn Leu Val Ser Glu His Ile Trp Cys Glu Asp
   835 840 845
   Phe Leu Val Arg Ser Phe Tyr Leu Lys Asn Val Gln Thr Gln Glu Thr
   850 855 860
   Arg Thr Leu Thr Gln Phe His Phe Leu Ser Trp Pro Ala Glu Gly Thr
   865 870 875 880
   Pro Ala Ser Thr Arg Pro Leu Leu Asp Phe Arg Arg Lys Val Asn Lys
   885 890 895
   Cys Tyr Arg Gly Arg Ser Cys Pro Ile Ile Val His Cys Ser Asp Gly
   900 905 910
   Ala Gly Arg Thr Gly Thr Tyr Ile Leu Ile Asp Met Val Leu Asn Arg
   915 920 925
   Met Ala Lys Gly Val Lys Glu Ile Asp Ile Ala Ala Thr Leu Glu His
       930                 935                 940
   Val Arg Asp Gln Arg Pro Gly Leu Val Arg Ser Lys Asp Gln Phe Glu
   945                 950                 955                 960
   Phe Ala Leu Thr Ala Val Ala Glu Glu Val Asn Ala Ile Leu Lys Ala
                   965                 970                 975
   Leu Pro Gln
<210> 2
   <211> 380
   <212> PRT
   <213> Homo sapiens
<400> 2
   Val Arg Gln His Ala Arg Gln Gln Asp Lys Glu Arg Leu Ala Ala Leu
   1               5                   10                  15
   Gly Pro Glu Gly Ala His Gly Asp Thr Thr Phe Glu Tyr Gln Asp Leu
               20                  25                  30
   Cys Arg Gln His Met Ala Thr Lys Ser Leu Phe Asn Arg Ala Glu Gly
           35                  40                  45
   Pro Pro Glu Pro Ser Arg Val Ser Ser Val Ser Ser Gln Phe Ser Asp
       50                  55                  60
   Ala Ala Gln Ala Ser Pro Ser Ser His Ser Ser Thr Pro Ser Trp Cys
   65                  70                  75                  80
   Glu Glu Pro Ala Gln Ala Asn Met Asp Ile Ser Thr Gly His Met Ile
                   85                  90                  95
   Leu Ala Tyr Met Glu Asp His Leu Arg Asn Arg Asp Arg Leu Ala Lys
               100                 105                 110
   Glu Trp Gln Ala Leu Cys Ala Tyr Gln Ala Glu Pro Asn Thr Cys Ala
           115                 120                 125
   Thr Ala Gln Gly Glu Gly Asn Ile Lys Lys Asn Arg His Pro Asp Phe
       130                 135                 140
   Leu Pro Tyr Asp His Ala Arg Ile Lys Leu Lys Val Glu Ser Ser Pro
   145                 150                 155                 160
   Ser Arg Ser Asp Tyr Ile Asn Ala Ser Pro Ile Ile Glu His Asp Pro
   165 170 175
   Arg Met Pro Ala Tyr Ile Ala Thr Gln Gly Pro Leu Ser His Thr Ile
   180 185 190
   Ala Asp Phe Trp Gln Met Val Trp Glu Ser Gly Cys Thr Val Ile Val
   195 200 205
   Met Leu Thr Pro Leu Val Glu Asp Gly Val Lys Gln Cys Asp Arg Tyr
   210 215 220
   Trp Pro Asp Glu Gly Ala Ser Leu Tyr His Val Tyr Glu Val Asn Leu
   225 230 235 240
   Val Ser Glu His Ile Trp Cys Glu Asp Phe Leu Val Arg Ser Phe Tyr
   245 250 255
   Leu Lys Asn Val Gln Thr Gln Glu Thr Arg Thr Leu Thr Gln Phe His
   260 265 270
   Phe Leu Ser Trp Pro Ala Glu Gly Thr Pro Ala Ser Thr Arg Pro Leu
   275 280 285
   Leu Asp Phe Arg Arg Lys Val Asn Lys Cys Tyr Arg Gly Arg Ser Cys
   290 295 300
   Pro Ile Ile Val His Cys Ser Asp Gly Ala Gly Arg Thr Gly Thr Tyr
   305 310 315 320
   Ile Leu Ile Asp Met Val Leu Asn Arg Met Ala Lys Gly Val Lys Glu
   325 330 335
   Ile Asp Ile Ala Ala Thr Leu Glu His Val Arg Asp Gln Arg Pro Gly
   340 345 350
   Leu Val Arg Ser Lys Asp Gln Phe Glu Phe Ala Leu Thr Ala Val Ala
   355 360 365
   Glu Glu Val Asn Ala Ile Leu Lys Ala Leu Pro Gln
   370 375 380
<210> 3
   <211> 576
   <212> PRT
   <213> Homo sapiens
<400> 3
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
               180                 185                 190
   Gln Pro Pro His Pro Ser Leu Ser Tyr Glu Pro Ala Leu Leu Gln Pro
           195                 200                 205
   Tyr Leu Phe His Gln Phe Gly Ser Arg Asp Gly Ser Arg Val Ser Glu
       210                 215                 220
   Gly Ser Pro Gly Met Val Ser Val Gly Pro Leu Pro Lys Ala Glu Ala
   225                 230                 235                 240
   Pro Ala Leu Phe Ser Arg Thr Ala Ser Lys Gly Ile Phe Gly Asp His
   245 250 255
   Pro Gly His Ser Tyr Gly Asp Leu Pro Gly Pro Ser Pro Ala Gln Leu
   260 265 270
   Phe Gln Asp Ser Gly Leu Leu Tyr Leu Ala Gln Glu Leu Pro Ala Pro
   275 280 285
   Ser Arg Ala Arg Val Pro Arg Leu Pro Glu Gln Gly Ser Ser Ser Arg
   290 295 300
   Ala Glu Asp Ser Pro Glu Gly Tyr Glu Lys Glu Gly Leu Gly Asp Arg
   305 310 315 320
   Gly Glu Lys Pro Ala Ser Pro Ala Val Gln Pro Asp Ala Ala Leu Gln
   325 330 335
   Arg Leu Ala Ala Val Leu Ala Gly Tyr Gly Val Glu Leu Arg Gln Leu
   340 345 350
   Thr Pro Glu Gln Leu Ser Thr Leu Leu Thr Leu Leu Gln Leu Leu Pro
   355 360 365
   Lys Gly Ala Gly Arg Asn Pro Gly Gly Val Val Asn Val Gly Ala Asp
   370 375 380
   Ile Lys Lys Thr Met Glu Gly Pro Val Glu Gly Arg Asp Thr Ala Glu
   385 390 395 400
   Leu Pro Ala Arg Thr Ser Pro Met Pro Gly His Pro Thr Ala Ser Pro
   405 410 415
   Thr Ser Ser Glu Val Gln Gln Val Pro Ser Pro Val Ser Ser Glu Pro
   420 425 430
   Pro Lys Ala Ala Arg Pro Pro Val Thr Pro Val Leu Leu Glu Lys Lys
   435 440 445
   Ser Pro Leu Gly Gln Ser Gln Pro Thr Val Ala Gly Gln Pro Ser Ala
   450 455 460
   Arg Pro Ala Ala Glu Glu Tyr Gly Tyr Ile Val Thr Asp Gln Lys Pro
   465 470 475 480
   Leu Ser Leu Ala Ala Gly Val Lys Leu Leu Glu Ile Leu Ala Glu His
   485 490 495
   Val His Met Ser Ser Gly Ser Phe Ile Asn Ile Ser Val Val Gly Pro
               500                 505                 510
   Ala Leu Thr Phe Arg Ile Arg His Asn Glu Gln Asn Leu Ser Leu Ala
           515                 520                 525
   Asp Val Thr Gln Gln Ala Gly Leu Val Lys Ser Glu Leu Glu Ala Gln
       530                 535                 540
   Thr Gly Leu Gln Ile Leu Gln Thr Gly Val Gly Gln Arg Glu Glu Ala
   545                 550                 555                 560
   Ala Ala Val Leu Pro Gln Thr Ala His Ser Thr Ser Pro Met Arg Ser
                   565                 570                 575
<210> 4
   <211> 200
   <212> PRT
   <213> Homo sapiens
<400> 4
   Met Arg Arg Pro Arg Arg Pro Gly Gly Leu Gly Gly Ser Gly Gly Leu
   1               5                   10                  15
   Arg Leu Leu Leu Cys Leu Leu Leu Leu Ser Ser Arg Pro Gly Gly Cys
               20                  25                  30
   Ser Ala Val Ser Ala His Gly Cys Leu Phe Asp Arg Arg Leu Cys Ser
           35                  40                  45
   His Leu Glu Val Cys Ile Gln Asp Gly Leu Phe Gly Gln Cys Gln Val
       50                  55                  60
   Gly Val Gly Gln Ala Arg Pro Leu Leu Gln Val Thr Ser Pro Val Leu
   65                  70                  75                  80
   Gln Arg Leu Gln Gly Val Leu Arg Gln Leu Met Ser Gln Gly Leu Ser
                   85                  90                  95
   Trp His Asp Asp Leu Thr Gln Tyr Val Ile Ser Gln Glu Met Glu Arg
               100                 105                 110
   Ile Pro Arg Leu Arg Pro Pro Glu Pro Arg Pro Arg Asp Arg Ser Gly
           115                 120                 125
   Leu Ala Pro Lys Arg Pro Gly Pro Ala Gly Glu Leu Leu Leu Gln Asp
       130                 135                 140
   Ile Pro Thr Gly Ser Ala Pro Ala Ala Gln His Arg Leu Pro Gln Pro
   145                 150                 155                 160
   Pro Val Gly Lys Gly Gly Ala Gly Ala Ser Ser Ser Leu Ser Pro Leu
                   165                 170                 175
   Gln Ala Glu Leu Leu Pro Pro Leu Leu Glu His Leu Leu Leu Pro Pro
               180                 185                 190
   Gln Pro Pro His Pro Ser Leu Ser
           195                 200
<210> 5
   <211> 173
   <212> PRT
   <213> Homo sapiens
<400> 5
   Met Gln His Pro Leu Trp Pro Gly Ser Ser Glu Gly Leu Gln Leu Leu
   1               5                   10                  15
   Val Cys Phe Leu Leu Leu Asn Ser Cys Pro Gly Gly Cys Ser Asp Thr
               20                  25                  30
   Ser Ala His Asp Gly Gln Asp Gln Val Gly Val Gly Gln Leu Trp Pro
           35                  40                  45
   Leu Gln Gly Phe Ala Thr Pro Val Phe Gln His Leu Gln Val Val Leu
       50                  55                  60
   Gln Gln Ile Ile Pro Gln Gly Leu Phe Trp Lys Asp Asp Ile Thr Gln
   65                  70                  75                  80
   Asp Ala Met Ile Gln Lys Met Glu His Ala Ser Arg Leu His Pro Gln
                   85                  90                  95
   Glu Pro Cys Leu Lys Asp Gly Lys Ala Leu Phe Pro Thr Lys Thr Thr
               100                 105                 110
   Glu Gln Glu Glu Lys Leu Gln Leu Leu Phe Pro Ser Glu Thr His Ser
           115                 120                 125
   Pro Leu Ala Lys Val Asn Arg Asp Gln Cys Phe Thr Ser Glu Val Val
       130                 135                 140
   Ser Lys Ala Leu Lys Gln Glu Val Ala Asn Pro Val Lys Gly Phe Ser
   145                 150                 155                 160
   Gly Pro Leu Pro Thr Val Gly Arg Asn Pro Val Ala Asp
   165 170
<210> 6
   <211> 14
   <212> PRT
   <213> Homo sapiens
<400> 6
   Asp Arg Val Tyr Ile His Pro Phe His Leu Leu Val Tyr Ser
   1 5 10
<210> 7
   <211> 8
   <212> PRT
   <213> Homo sapiens
<400> 7
   Asp Arg Val Tyr Ile His Pro Phe
   1 5
<210> 8
   <211> 2438
   <212> DNA
   <213> Homo sapiens
<400> 8
   aaacatgggg cggggcggcg cggccgggga agcgtgatga aggcctacga gtgcggcgcg 60
   gcctgaaggg gcacgcgggg gacctgcaaa gctagtgagg ggcggggcag gcggcgcggt 120
   gggggcgggc cgagcccgga ggccagatga gcggacacag ccccacgcgc ggggccatgc 180
   aggtggccat gaacggtaag gcccgcaaag aggcggtgca gactgcggct aaggaactcc 240
   tcaagttcgt gaaccggagt ccctctcctt tccatgctgt ggctgaatgc cgcaaccgcc 300
   ttctccaggc tggcttcagt gaactcaagg agactgagaa atggaatatt aagcccgaga 360
   gcaagtactt catgaccagg aactcctcca ccatcatagc ttttgctgta gggggccagt 420
   acgttcctgg caatggcttc agcctcatcg gggcccacac ggacagcccc tgcctccggg 480
   tgaaacgtcg gtctcgccgc agccaggtgg gcttccagca agtcggtgtg gagacctatg 540
   gtggtgggat ctggagcacc tggtttgacc gtgacctgac tctggctgga cgcgtcattg 600
   tcaagtgccc tacctcaggt cggctggagc agcagctggt gcacgtggag cggcccattc 660
   ttcgcatccc acacctggcc atccatctgc agcgaaatat caacgagaac tttgggccca 720
   acacagagat gcatctagtc cccattcttg ccacagccat ccaggaggag ctggagaagg 780
   ggactcctga gccagggcct ctcaatgctg tggatgagcg gcaccattcg gtcctcatgt 840
   ccctgctctg tgcccatctg ggctgagccc ccaaggacat agtggagatg gagctctgcc 900
   ttgcagacac ccagcctgcg gtcttgggtg gtgcctatga tgagttcatc tttgctcctc 960
   ggctggacaa tctgcacagc tgcttctgtg ccctgcaggc cttgatagat tcctgtgcag 1020
   gccctggctc cctggccaca gagcctcacg tgcgcatggt cacactctat gacaacgaag 1080
   aggtggggtc tgagagtgca cagggagcac agtcactgct gacagagctg gtgctgcggc 1140
   ggatctcagc ctcgtgccag cacccgacag ccttcgagga agccataccc aagtccttca 1200
   tgatcagcgc agacatggcc catgctgtgc atcccaacta cctggacaag catgaggaga 1260
   accaccggcc tttattccac aagggccccg tgatcaaggt gaacagcaag caacgctatg 1320
   cttcaaacgc ggtgtcagag gccctgatcc gagaggtggc caacaaagtc aaggtccccc 1380
   tgcaggatct catggtccgg aatgacaccc cctgtggaac caccattgga cctatcttgg 1440
   cttctcggct ggggctgcgg gtgctggatt taggcagccc ccaactggcc atgcactcta 1500
   tccgggagat ggcctgcacc acaggagtcc tccagaccct caccctcttc aagggcttct 1560
   ttgagctgtt cccttctcta agccataatc tcttagtgga ttgagccctc ttggaaagac 1620
   ttctctgcca tccctttgca cctgagaggg gaagttctca gctgagctga agctggatta 1680
   ttaaagtgga ttgtcactca gactctccgt gctacgctta tttggagact agaggagtgg 1740
   gagttgagcc tggcttgaac ctttggaacc agaaaagttg gggagcaggt ggaggaggcc 1800
   acactcctgg gagctgatgg ttttaaatct ggttttaaat ctcttctctg tctcaagtcc 1860
   atgtctgaag tgggtgaagg gtggactgga tcctcaagca gaaggtcact tctcccaccc 1920
   ctagtcctcc acctgggaaa tggcctcaac ggtcttccct ctttccatcc ccagaatggg 1980
   tgtcccgctc tgccttcaga gatcctgttc tgcactgggc caccttcaga gctgctctgg 2040
   gcagacaaag ctgtttctca ccctcccaaa atcccttccc atcccctctc aggagctgca 2100
   aagagaaggg cacagtctat accttatcac ccttcatcat ccacttctca gcagtccctg 2160
   gtattacttg attgtgaaca tattatcagg aaaaaagatt atcattgaaa aaattggaaa 2220
   agtcaaagtc tggccgggtg cagcggctca catctgtatt cccagcattt tgggaggcca 2280
   aggcaggtgg atcacttgag gtcagagttc aagaccagcc ttgccaacat ggtgaaaccc 2340
   atctctactt aaaaaaaaat acaaaaaaat tagccaggca tggtggcaca cacctgtaat 2400
   ctcagctatt caggagactg aggcaggaga atagcttg 2438
<210> 9
   <211> 485
   <212> PRT
   <213> Homo sapiens
<400> 9
<210> 10
   <211> 602
   <212> DNA
   <213> Homo sapiens
<400> 10
   gaattcattt attaaaacag aacacatagg ggtttaatca atatccttaa attttccaca 60
   aacataacat aaataaactc cacgttgtga ggaagagagg atttttaata catatgtgtt 120
   gaatgaatga tcattattta gataaatgaa tgactgaagt gattgttata ttcaggtaaa 180
   ttcatcatgg ctaggtagca aaccaaagac ttgtaagaac ctcaaatgag gacatgcaca 240
   aaacagggat ggccatgggc tacgtaattt caaggtcttt tgtcttcaac gtcaaaattc 300
   actttagaga acttaagtga ttttcatgcg tgattgaaag tagaaggtgg atttccaagc 360
   tgctctctcc attcccaacc aaaaaaaaaa aaaaaagata caagagtgca taaaaagttt 420
   ccaggtctct aaggtctctc acccaatata agcatagaat gcagatgagc aaagtgagtg 480
   ggagagggaa gtcatttgta acaaaaactc attatttaca gatgagaaat ttatattgtc 540
   agcgtaatat ctgtgaggct aaacagagct ggagagtata taaaagcagt gcgccttggt 600
   gc 602
<210> 11
   <211> 21
   <212> DNA
   <213> Mus musculus
<400> 11
   agaggccatc caggccacat t 21
<210> 12
   <211> 1682
   <212> DNA
   <213> Mus musculus
<400> 12
   gagcgatcga gctaggaatc cacatctgtc gccagtgaag tcctcggaag cagagatggc 60
   tatgaacggc agggctcgga aagaggccat ccaggccaca gcccgagagc tcctgaagtt 120
   cgtgaaccgg agtccctctc ctttccacgt cgtggctgag tgccgcagcc gcctcctcca 180
   ggctggcttc cgtgaactca aggaaacaga gggctgggat atcgtaccag aaaacaagta 240
   cttcttaacc agaaactcct cctccatcat tgcttttgct gtggggggcc agtatgttcc 300
   tggcaatggc ttcagcctca ttggggccca cacggacagt ccctgtctca gggtgaaacg 360
   caaatcgcgc cgaagccagg tgggctacca ccaggtcggt gttgagacct acggcggtgg 420
   gatctggagc acatggttcg accgggacct gaccttggct ggacgcgtca ttatcaagtg 480
   ccctacctca ggccggctgg agcagaggct cgtccatata gaacggccca tcctgcgcat 540
   cccacatctg gccatccatc tgcagcgaaa tatcaatgag aactttgggc ccaacacaga 600
   gatccaccta gtccctattc ttgccacagc agttcaggaa gagctggaga aagggactcc 660
   tgaaccaggg cctctcggtg ccactgatga gcggcaccac tcggtcctca tgtccctgct 720
   ctgtacccat ctgggcctga gtcctgacag catcatggag atggagctct gcctggcaga 780
   cacccagcct gcagtcctgg gtggagccta tgaagagttc atcttcgctc ctcgactgga     840
   caatctgcac agctgcttct gtgccctgca ggcattgatt gattcctgtg catcccctgc     900
   ctctctggcc agggatccac atgtgcgcat ggtcacactc tacgacaatg aagaggtggg     960
   gtcagagagt gcacaaggag cacagtccct gctgacggag ctgatactac ggcgcatctc    1020
   agcctcacct cagcgtctga ctgccttcga ggaagccata cctaagtcct tcatgatcag    1080
   tgcagacatg gcccatgctg tgcacccaaa ctactcggac aagcatgaag aaaaccaccg    1140
   gcctttgttc cataagggtc ctgtgatcaa ggtgaacagc aagcagcgct acgcctctaa    1200
   tgcagtgtct gagtccatga ttcgagaggt ggctggccaa gttggggtgc ccctgcagga    1260
   cctcatggtc aggaatgact ccccgtgtgg taccaccatt ggacctatct tggcttctcg    1320
   actggggctt cgggtgctgg acttaggcag cccccaactg gctatgcact ctatccggga    1380
   gacagcctgt accactggag ttctccagac cctcacccct ttcaagggct tctttgagct    1440
   gttcccttct gtaagccgga acctcttagt ggactgaggc ctctcgccaa gagttgtctt    1500
   ccaccccttc aattgagagg ggaggatctc agtttcgctg atgttggtta ttaaagtaga    1560
   ttttcactcc aaaaaaaaaa aaaaaaaaaa aaaaaaaaaa aaaaaaaaaa aaaaaaaaaa    1620
   aaaaaaaaaa aaaaaaaaaa aaaaaaaaaa aaaaaaaaaa aaaaaaaaaa aaaaaaaaaa    1680
   aa                                                                   1682
<210> 13
   <211> 21
   <212> DNA
   <213> Mus musculus
<400> 13
   aatgtggcct ggatggcctc t 21

## Description

### BACKGROUND

Type 1 diabetes, often referred to as juvenile diabetes, is associated with islet cell dysfunction which results in loss of insulin production. Understanding how hormones such as insulin and glucagon are produced and regulated for secretion in islets of the pancreas is important for the prevention and treatment of type 1 diabetes.

Bioinformatic analysis and subsequent localization studies of clustered genes related to islet function can provide clues for further studies of their roles in glucose metabolism. Sequence analysis and chromosome mapping revealed that the Protein Tyrosine Phosphatase Receptor type N gene (*PTPRN*; gene ID number 5798) was clustered with Regulated Endocrine-Specific Protein 18 (*RESP18*) at human chromosome 2q35. G. Zhang et al., J. Endocrinol., 195, 313-321 (2007). Previous phylogenetic analysis showed that *PTPRN* and *RESP18* formed a conserved syntenic block in mammalian but not non-mammalian species. G. Zhang et al., J. Endocrinol., 195, 313-321 (2007).

PTPRN is a secretory granule membrane protein in islet cells and a major autoantigen in type 1 diabetes. *See* M.S. Lan et al., Proc. Natl. Acad. Sci. U. S. A., 93, 6367-6370 (1996); M. Solimena et al., EMBO J., 15, 2102-2114 (1996). PTPRN (SEQ ID NO.1) is composed of a cytoplasmic region (a.a. 600∼979; SEQ ID NO. 2) and a luminal region (a.a. 1∼576; SEQ ID NO. 3), which has significant homology at a.a. 1-200 (SEQ ID NO. 4) with RESP18 (SEQ ID NO. 5). G. Zhang et al., J. Endocrinol., 195, 313-321 (2007). Both *RESP18* and *PTPRN* were expressed in secretory granules of islet cells and transcriptionally upregulated upon high glucose stimulation or hyperglycemia. G. Zhang et al., J. Endocrinol., 195, 313-321 (2007). *PTPRN* is evolutionarily conserved from C. *elegans* to human (T. Cai et al., Diabetologia, 44, 81-88. (2001)) and mutation of the *PTPRN* orthologous gene in *C*. *elegans* has also impaired insulin-like pathway (T. Cai et al., J. Neurosci., 24, 3115-3124 (2004)). *Ptprn*-deficient mice showed reduced insulin secretion from isolated islets and glucose intolerance. K. Saeki et al., Diabetes, 51, 1842-1850 (2002).

PTPRN and RESP18 were also associated with secretory granules and granule-enriched fractions in the brain and endocrine cells. M. Solimena et al., EMBO J., 15, 2102-2114 (1996); DN Darlington et al., J. Histochem. Cytochem., 45, 1265-1277 (1997). *DNPEP,* another neighbor gene of *PTPRN,* encodes aspartyl aminopeptidase (DAP, EC 3.4.11.21), a mammalian homolog of the M18 family in yeast, which was believed to be a cytosolic protein with high enzymatic activity in neuroendocrine tissues. According to earlier research, DAP was believed to be a cytosolic enzyme associated with lysosome in mammals (S. Wilk et al., J. Biol. Chem., 273, 15961-70 (1998); S. Wilk et al., Arch. Biochem. Biophys., 407, 176-183 (2002)) or vacuoles in yeast (ND Rawlings et al., Biochem J., 290 (Pt 1), 205-218 (1993); Metz et al., Biochim. Biophys. Acta, 429, 933-949 (1976)). Mammalian DAP was reported to be preferentially expressed in neuron and neuroendocrine tissues and high activity of DAP was found in testis, kidney (S. Wilk et al., J. Biol. Chem., 273, 15961-70 (1998), and the myelinic and synaptosomal fraction of brain tissues (G. Larrinaga et al., Neurosci. Lett., 383, 136-140 (2005)). DAP was reported to convert angiotensin I to angiotensin 2-10 (MK Sim, Blood Press., 3, 260-264 (1994)) and also to degrade angiotensin II to angiotensin III (S. Wilk, J. Biol. Chem., 273, 15961-15970 (1998)), suggesting that DAP played a major role in blood pressure (I. Banegas, J. Renin Angiotensin Aldosterone Syst., 7, 129-134 (2006)).

PTPRN was recently found in kidney as well. Knockout of the *Ptprn* gene in mice resulted in a marked reduction of renin-angiotensin levels in plasma (SM Kim et al., Am. J. Physiol. Renal Physiol., 296, F382-389 (2009)), which suggests that DAP and PTPRN play a similar role in regulating blood pressure in kidney. The RESP18 protein was also found in the kidney tissue by Western blots (MR Schiller et al., J. Biol. Chem., 270, 26129-26138 (1995)). However, whether DAP is colocalized with PTPRN and/or RESP18 in the same cells of kidney needs further investigation.

The major renin-angiotensin system (RAS) components, including angiotensinogen, Angiotensin I (Ang I), Angiotensin II (Ang II), Angiotensin III (Ang III), Renin, and Angiotensin-Converting Enzyme (ACE), were found in islets and islet MIN6 cells. Ramracheya et al., Diabetologia, 49, 321-331 (2006); Leung et al., Adv. Exp. Med. Biol., 654, 339-361 (2010). The local RAS plays paracrine and autocrine roles in the regulation of various functions, such as cell proliferation, apoptosis, and hormone secretion, in islets. Angiotensinogen is only expressed in alpha cells and not in other islet cells. Regoli et al., J. Endocrinol., 179, 81-89 (2003). Ang II, known for its blood pressure maintenance and body fluid balance functions, has been implicated in the regulation of the local islet RAS. Beyond the role in the regulation of blood pressure, Ang II can also regulate insulin signaling, thus leading to insulin resistance and diabetes.

### SUMMARY

The methods provided herein are based on the discovery, as described in more detail herein, that aspartyl aminopeptidase (DAP) is expressed in mammalian pancreatic islet cells and that increased DAP expression results in decreased Angiotensin II (Ang II) levels in the islet cells. Conversely, it was also found that inhibition of DAP expression resulted in increased Ang II levels. It has not been previously reported that DAP was expressed in mammalian pancreatic islet cells or that DAP expression levels in the islet cells impacted intracellular or extracellular Ang II levels. The methods of the present invention are defined in the claims.

By one approach, a screening method is provided for identifying a compound effective to increase DAP activity in mammalian pancreatic islet cells, the method comprising: contacting purified DAP and Ang II with a test compound; measuring the level of Ang II; and determining if the level of Ang II is lower when treated with the test compound than an otherwise identical control containing DAP and Ang II that has not been contacted with the test compound. In one aspect, DAP and Ang II are contacted with the test compound for an amount of time effective for DAP to degrade at least about 10 percent of the Ang II present so that differences in activity between the test sample and the control sample can be detected. By this approach, compounds that are effective cofactors or enzyme activators can be identified.

In another aspect, a screening method is provided for identifying a compound that restrains, blocks, or suppresses a DAP inhibitor and thereby increases DAP activity in mammalian pancreatic islet cells upon inactivation of the inhibitor. The method comprises contacting an islet cell lysate with a test compound and purified DAP and Ang II; measuring the level of Ang II; and determining if the level of Ang II is lower when treated with the test compound than an otherwise identical control containing the cell lysate, DAP, and Ang II that has not been contacted with the test compound.

Once it has been determined that a DAP inhibitor is present in the lysate, a screening method is provided for identifying a compound effective to increase DAP expression in mammalian pancreatic islet cells, the method comprising: culturing mammalian pancreatic islet cells capable of producing DAP in the presence of a test compound; measuring the level of *DNPEP* gene expression; determining if the level of *DNPEP* gene expression is higher when treated with the test compound than an otherwise identical control not cultured with the test compound. The level of gene expression can be determined by measuring the amount of DAP and/or the amount of mRNA that encodes DAP. By another approach, the method may further comprise measuring the level of Ang II and determining if the level of Ang II is lower when treated with the test compound.

Treatment or prevention of type 1 diabetes could comprise
administering to the subject a therapeutically effective amount of a test compound identified according to the methods herein. It is also presently believed that reducing the Ang II content of the pancreatic islet cells may be effective at controlling insulin resistance and, hence, type II diabetes.

In yet another aspect, a method is provided for increasing DAP expression and decreasing Ang II levels, the method comprising delivering one or more copies of a polynucleotide encoding DAP to pancreatic islet cells. By one approach, the one or more copies of a polynucleotide encoding DAP can be delivered via a viral vector.

### BRIEF DESCRIPTION OF THE DRAWINGS

The foregoing features, as well as other features, will become apparent with reference to the description and Figures below.

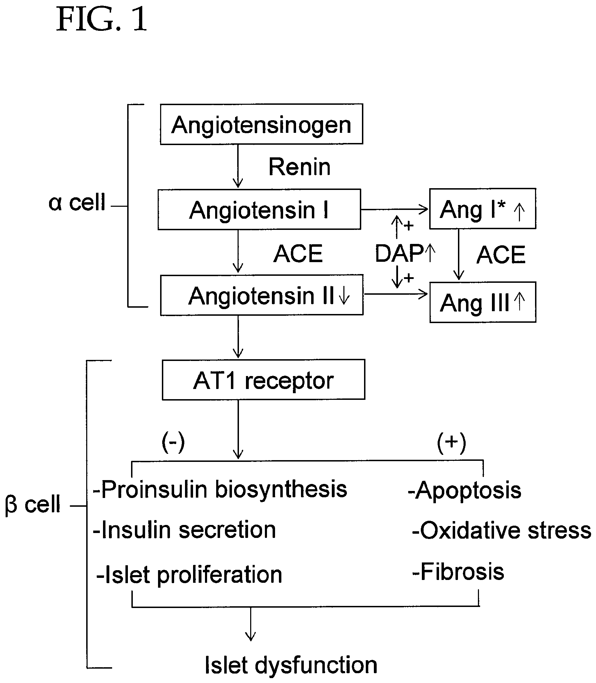

FIG. 1 is a schematic showing DAP and the islet renin-angiotensin system (RAS) metabolism in islet cells. The major RAS components, including angiotensinogen, Ang I, Ang II, Ang III, Renin, and ACE, were found in islets and MIN6 cells. ("+" indicates activation or increase; "-" indicates inhibition or decrease.)

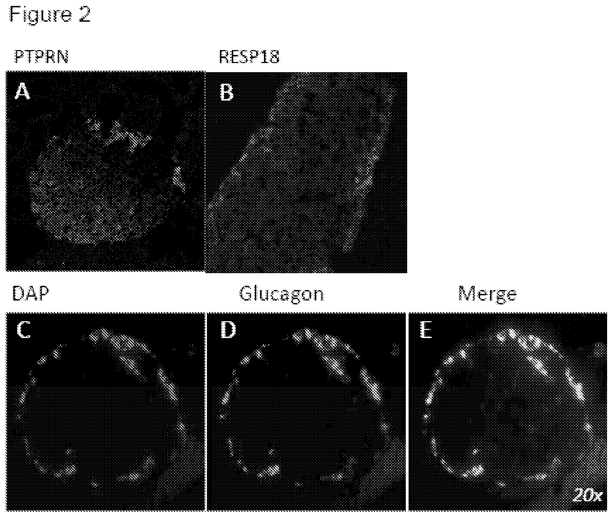

FIGS. 2A-E are microscope images taken with a Zeiss Axiophot microscope, which illustrates how PTPRN (FIG. 2A), RESP18 (FIG. 2B); DAP (FIG. 2C), and glucagon (FIG. 2D) are expressed in islet cells. All images are presented in 20× amplification. PTPRN (FIG. 2A) and are expressed in pancreatic islet cells with stronger signal in peripheral alpha cells. DAP (FIG. 2C) has a similar expression pattern to that of glucagon-positive cells (FIG. 2D), and is completely overlapped with the glucagon signal (FIG. 2E), as determined by double stainings with antibodies to DAP and glucagon separately.

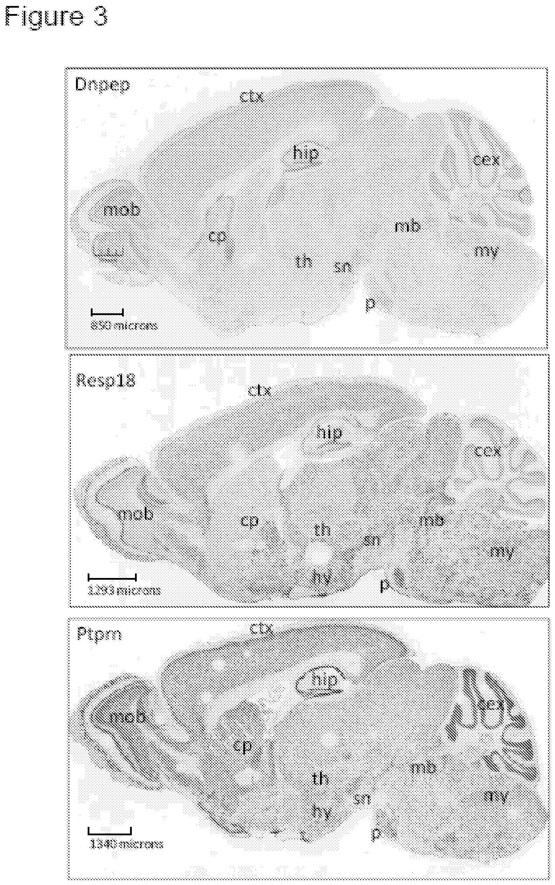

FIG. 3 is representative images of *in situ* hybridization in the adult mouse brain (sagittal). Scale bars are indicated in microns. mRNAs of *Dnpep, Resp18* and *Ptprn* are detected with a similar pattern in various brain regions, including midbrain (mb), medulla (my), pons (p), thalamus (th), subthalamic nucleus (sn), hippocampal region (hip), main olfactory bulb (mob), cerebral cortex (ctx), caudate putamen (cp), and cerebellum (ctx) as judged by staining in a sagittal section of mouse brain from the Allen Brain Atlas.

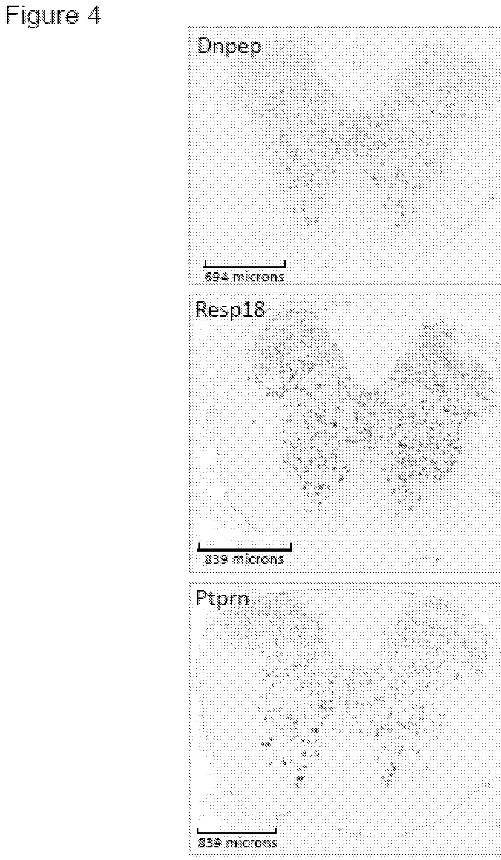

FIG. 4 are representative *in situ* expression images of *Dnpep, Resp18* and *Ptprn* mRNAs in cervical spinal cord of adult mice. Scale bars are indicated in microns. All of the three genes are mainly expressed in gray matter that is shaped like a butterfly which contains interneurons and motor neurons.

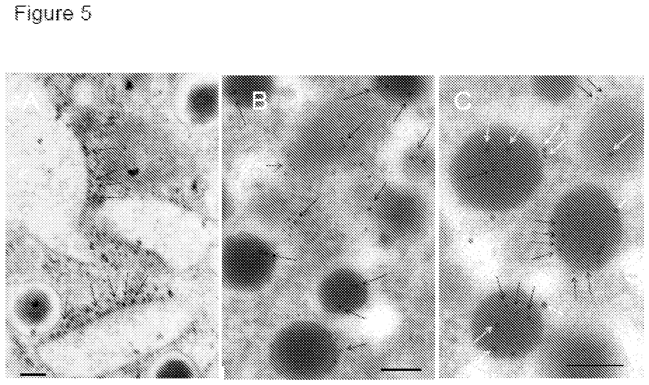

FIGS. 5A-C are electron micrographs showing subcellular distribution of DAP in islet alpha cells. Scale bars are 100 nm. The anti-DAP rabbit antibody was detected with goat anti-rabbit IgG antibody conjugated to colloidal gold. (A) DAP particles are abundantly located around lysosomal-like structure (10 nm colloidal gold) and are also detected in the luminal region of secretory granules (B, 5 nm colloidal gold) as indicated by arrows. DAP particles were not detected in mitochondria or nuclei. (C) DAP (5 nm colloidal gold particles, black arrows) is colocalized with glucagon (detected by 10 nm colloidal gold particles conjugated to goat anti-mouse antibody, white arrows) in DCVs.

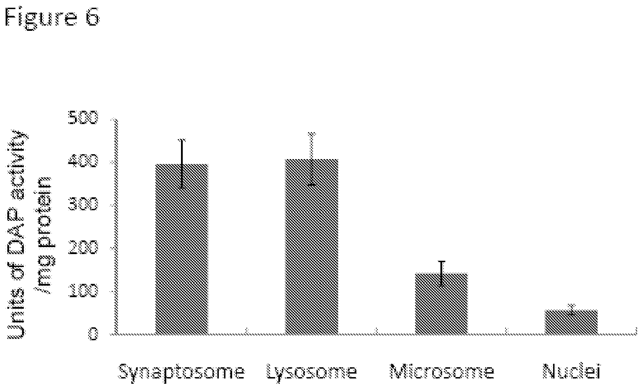

FIG. 6 is a bar graph illustrating subcellular distribution of DAP enzymatic activities in mouse brain. Values were expressed as units of aminopeptidase per mg protein from three independent experiments (Mean ± S.E.M).

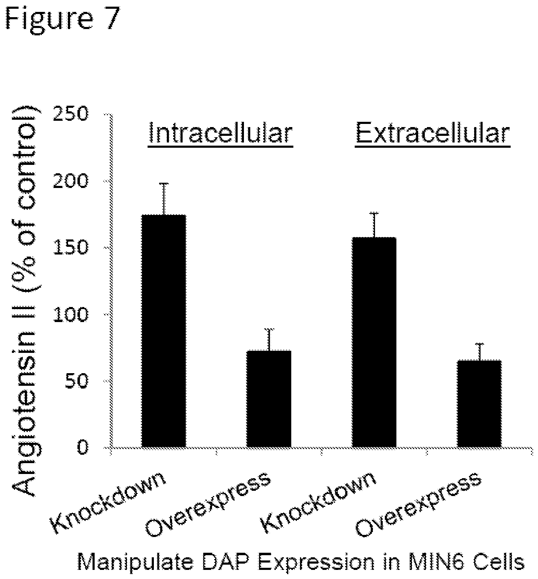

FIG. 7 is a bar graph illustrating the effects of DAP expression on angiotensin II (Ang II) levels in islet MIN6 cells.

### DETAILED DESCRIPTION

The methods provided herein are based on the discovery, as described in more detail herein, that aspartyl aminopeptidase (DAP) is expressed in mammalian pancreatic islet cells and that increased DAP expression results in decreased Ang II levels in the islet cells. Conversely, it was also found that inhibition of DAP expression resulted in increased Ang II levels. It was not previously reported that DAP was expressed in mammalian pancreatic islet cells or that DAP expression levels in the islet cells impacted intracellular and extracellular Ang II levels.

Many homologous genes are clustered in genomes and evolutionarily conserved in syntenic segments cross-species. *DNPEP* is an evolutionarily conserved gene found from *C*. *elegans* to human. It was discovered that *DNPEP* was physically linked with *PTPRN* in genomes in both human and *C*. *elegans,* suggesting a strong syntenic conservation of the block over evolutionary spectrum of bilaterian animals. Conserved genes within the same syntenic block may keep similar tissue specific expression patterns in neuroendocrine tissues including islets and neuronal tissues in the brain and spinal cord and hence may also be involved in similar biological functions.

Through genomic localization, tissue specific expression, subcellular localization and enzyme activity studies presented herein, it was demonstrated that DAP and Protein Tyrosine Phosphatase Receptor type N (PTPRN) are colocalized in the secretory granules of pancreatic islet cells. It was also found that DAP was colocalized with glucagon in hormone secretory granules in pancreatic islet cells. *In situ* hybridization analysis showed very similar expression patterns of *Dnpep, Resp18* and *Ptprn* in both the brain and spinal cord. Since genes expressed in the same tissue and cellular localization may have similar regulatory elements in their highly conserved noncoding elements (HCNEs), identification of such genomic regulatory blocks (GRBs) with specific expression patterns in pancreatic endocrine cells may provide clues to promote further investigation in corresponding biological functions of these genes. These findings suggest that genes from this syntenic block may also function coordinately. This colocalization signifies that DAP may play an important role in posttranslational processing and secretion of hormones.

Since the 5'-terminal flanking region of *DNPEP* showed considerable similarity to that of *PTPRN* and *RESP18,* it appeared that the expression of these genes may be regulated by common transcription factor(s) for their specific expression in islet cells. It was also found that DAP was expressed in mouse and human islet alpha cells and was subcellularly associated with secretory granules and lysosomal-like compartments. DAP enzymatic activity assay demonstrated that the highest activities of DAP was detected in synaptosomal- and lysosomal-enriched cellular fractions of brain tissues. These findings support the notion that genes located in the same syntenic block may also share similar expression patterns and even related biological functions.

As described further in the Examples herein, it was found that upregulated DAP expression decreases the intracellular and extracellular Ang II levels by deleting the N-terminal aspartic (Asp) of both Ang I (1-14) (Asp-Arg-Val-Tyr-Ile-His-Pro-Phe-His-Leu-Leu-Val-Tyr-Ser; SEQ ID NO. 6) and Ang II (1-8) (Asp-Arg-Val-Tyr-Ile-His-Pro-Phe; SEQ ID NO. 7), which provides Ang I* (des-Asp-Ang I) and Ang III (des-Asp-Ang II), respectively. Ang I appears to have no biologically activity and exists solely as a precursor to Ang II. Ang II can regulate insulin signaling, thus leading to insulin resistance and diabetes through a complicated process as shown in FIG. 1. After being processed by renin and angiotensin I converting enzyme (ACE), Ang II is secreted and binds to the type-1 angiotensin receptor (AT1) on beta cells, which activates downstream JAK2-STAT pathway. In pathological conditions, over-activation of Ang II and AT1 receptor results in islet beta cell dysfunction and diabetes. Accordingly, DAP can be targeted by potential small molecules to prevent or control type 1 diabetes.

The discovery of the relationship between the expression of DAP, the gene product of *DNPEP,* and Ang II levels in mammalian pancreatic islet cells can be utilized to identify compounds capable of modulating DAP levels in the islet cells. In one aspect, a method is provided for identifying a compound that increases the expression of DAP, the gene product of *DNPEP,* in mammalian pancreatic islet cells. In another aspect, a method is provided for identifying a compound that increases DAP activity in mammalian pancreatic islet cells. In both aspects, the increased DAP expression or activity results in decreased angiotensin II (Ang II) levels in the mammalian pancreatic islet cells, thereby preventing Ang II induced pancreas islet cell dysfunction and preventing or reversing diabetes.

In another aspect, a method can then be provided for treating or preventing type 1 diabetes by administering a pharmaceutical composition comprising an effective amount of the identified compound which is effective to increase the expression or activity of DAP in mammalian pancreatic islet cells and thereby decreasing Ang II levels.

### Terms

The term "compound" is used in the context of a "test compound" or a "drug candidate compound" described in connection with the assays described herein. Test compounds include, for example, peptides, proteins, antibodies, non-peptide compounds, synthetic compounds, fermentation products, cell extracts, plant extracts, animal tissue extracts, plasma and the like. As such, these compounds comprise organic or inorganic compounds, derived synthetically or from natural sources. In one aspect, libraries of compounds can be used for high-throughput purposes. Suitable libraries include, for example, antibody fragment libraries, lipid libraries, synthetic compound libraries, natural compound libraries, and peptide libraries. Other libraries may be used, if desired. The compounds also include pharmaceutically acceptable salts thereof.

The term "contact" or "contacting" means bringing at least two moieties together, including both *in vitro* and *in vivo* systems.

The term "condition" or "disease" means the presentation of symptoms (i.e., illness) or the manifestation of abnormal clinical indicators (e.g., biochemical indicators), resulting from islet cell dysfunction, including type I diabetes. Alternatively, the term "disease" refers to a genetic or environmental risk of or propensity for developing such symptoms or abnormal clinical indicators.

The term "effective amount" or "therapeutically effective amount" means that amount of a compound that will elicit the biological or medical response of a subject that is being sought by a medical doctor or other clinician. In one aspect, particularly with regard to treating type 1 diabetes, the term "effective amount" is intended to mean the amount of a compound or agent that will bring about a biologically meaningful decrease in the levels of Ang II in the subject's islet cells.

The term "expression" comprises both endogenous expression and overexpression by transfection.

The terms "substantially pure" or "substantially purified" as used herein in reference to a given polypeptide means that the polypeptide is substantially free from other biological macromolecules. For example, the substantially pure polypeptide is at least about 70 percent, in another aspect at least about 75 percent, in another aspect at least 80 percent, in another aspect at least about 85 percent, in another aspect at least about 90 percent, and in yet another aspect at least about 95 percent pure by dry weight. Purity can be measured by any appropriate standard method known in the art, such as, but not limited to, column chromatography, polyacrylamide gel electrophoresis, or HPLC analysis. For use in these experiments, the polypeptides used herein, such as DAP and/or Ang II, can be purified using known techniques, including, for example, salt precipitation, differential solubilization, chromatography (e.g., high performance liquid chromatography, ion exchange chromatography, gel permeation chromatography, hydrophobic interaction chromatography, affinity chromatography, and immunoaffinity chromatography), centrifugation, and combinations thereof.

The term "subject" includes mammals and specifically includes humans.

The term "treating" means an intervention performed with the intention of preventing the development or altering the pathology of, and thereby alleviating a disorder, disease or condition, including one or more symptoms of such disorder or condition. Accordingly, "treating" refers to both therapeutic treatment and prophylactic or preventative measures. The related term "treatment," as used herein, refers to the act of treating a disorder, symptom, disease or condition, as the term "treating" is defined above. Those in need of treatment include those already with the disorder as well as those in which the disorder is to be prevented.

The term "Angiotensin II" or "Ang II" as used herein includes a protein having the amino acid sequence of SEQ ID NO: 7, or a polypeptide in which one or more amino acids is modified by deletion, addition, and/or substitution by another amino acid, wherein said protein is functionally equivalent to the polypeptide having the amino acid sequence of SEQ ID NO: 7.

The nucleotide sequence of the DAP cDNA and the amino acid sequence of the protein encoded by the cDNA are shown in SEQ ID NOs: 8 and 9, respectively. The term "aspartyl aminopeptidase" or "DAP" as used herein includes a protein having the amino acid sequence of SEQ ID NO: 9, or a protein in which one or more amino acids is modified by deletion, addition, and/ or substitution by another amino acid, wherein said protein is functionally equivalent to the protein having the amino acid sequence of SEQ ID NO: 9. In one aspect, the protein has at least 80 percent identity to the amino sequence of SEQ ID NO: 9, in another aspect at least 85 percent identity, in another aspect at least 90 percent identity, and in yet another aspect at least 95 percent identity. It is known in the art that a protein may have an amino acid sequence which is modified by deletion, addition, and/or substitution by other amino acids yet still retain its biological activity.

The hybridization technique (Sambrook et al., Molecular Cloning 2nd ed. 9.47-9.58, Cold Spring Harbor Lab. press, 1989) is well known to those of skill in the art as an alternative to preparing a protein functionally equivalent to a certain protein. More specifically, one skilled in the art can utilize the general procedure to obtain a protein functionally equivalent to human DAP by isolating DNA having a high homology with the whole or part of the cDNA (SEQ ID NO: 8) encoding human DAP. Accordingly, the term "DAP" as used herein includes such proteins that are encoded by DNA that hybridizes with cDNA encoding human DAP or part thereof and that are functionally equivalent to a human DAP. For instance, homologues of human DAP in other mammals (such as those of monkey, mouse, rabbit, and bovine) are included.

Stringent hybridization conditions for isolating DNA encoding functionally equivalent proteins of human DAP can be suitably selected by one skilled in the art, and for example, low-stringent conditions can be given. Low-stringent conditions are, for example, 42°C, 2×SSC, and 0.1% SDS, and in another aspect, 50°C, 2×SSC, and 0.1% SDS. In some aspects, highly stringent conditions are more preferable and include, for example, 65°C, 2×SSC, and 0.1% SDS. Under these conditions, the higher the temperature, the higher the homology of the obtained DNA. However, several factors other than temperature, such as salt concentration, can influence the stringency of hybridization and one skilled in the art can suitably select the factors to accomplish a similar stringency.

Alternatively, a gene amplification method, for example, the polymerase chain reaction (PCR) method, can be utilized to isolate the object DNA using primers synthesized based on the sequence information of the DNA encoding human DAP (SEQ ID NO: 8). One of ordinary skill in the art of design of amplification primers will recognize that a given primer need not hybridize with 100 percent complementarity in order to effectively prime the synthesis of a complementary nucleic acid strand in an amplification reaction. Moreover, a primer may hybridize over one or more segments such that intervening or adjacent segments are not involved in the hybridization event (e.g., a loop structure or a hairpin structure). In one aspect, the primers are at least 13 nucleotides in length. In another aspect, the primers are less than 36 nucleotides in length. In some aspects, the function of a given primer may be substituted by a combination of two or more primers that hybridize adjacent each other or that are linked by a nucleic acid loop structure or linker which allows a polymerase to extend the two or more primers in an amplification reaction.

### Screening Method for Identifying a Compound that Increases DAP Activity, Directly or Indirectly, in Mammalian Pancreatic Islet Cells

By one approach, methods are provided for identifying a compound which is effective to increase the activity of DAP, either directly or indirectly, in mammalian pancreatic islet cells. By "increased activity" is meant that the amount of Ang II degraded by DAP per unit of time is higher in the presence of the compound while the amount of DAP, temperature, pH, and other environmental variables are held constant. In one aspect, activity is increased by at least about 10 percent, in another aspect at least about 20 percent, in another aspect at least about 30 percent, in another aspect at least about 40 percent, in another aspect at least about 50 percent, in another aspect at least about 60 percent, and in yet another aspect at least about 75 percent. A compound may be effective to increase DAP activity by several mechanisms as discussed below.

In one aspect, the identified compound may be a cofactor that is necessary for enzymatic activity of DAP. Cofactors may include any non-protein compound, such as, for example, organic molecules, such as coenzymes and prosthetic groups, or inorganic molecules, such as metallic cations. In some aspects, at least one cofactor may be needed for the enzymatic activity of DAP. While the cofactor may be found in the pancreatic islet cells, the cofactor may be present in an insufficient quantity such that the amount present in the cells limits DAP activity. For example, the cofactor may be present in limited quantity in diseased cells. Therefore, providing increased amounts of cofactor to the islet cells is expected to be effective to increase DAP activity.

In another aspect, the identified compound may be an enzyme activator that is effective to increase DAP activity. An activator is a substance other than a substrate or cofactor that is effective to increase the rate of the enzymatic reaction.

In another aspect, the identified compound may be effective to increase DAP activity indirectly by interfering with an inhibitor of DAP activity. For example, the compound may interfere by restraining, blocking, or suppressing the DAP inhibitor. An enzyme inhibitor is a molecule that decreases the enzyme's activity after binding to the enzyme. The inhibitor's interaction with the enzyme can be reversible or irreversible. There may be one or more inhibitors of DAP in the islet cells which are involved in the regulation of DAP activity in the cells. For example, the inhibitor may be involved in a negative feedback loop where accumulation of a downstream product inhibits future production by the system. Such negative feedback mechanisms are known to be involved in the regulation of many types of hormones. In some cases, the inhibitor may bind to DAP in its active site or may bind to another portion of DAP, such as a regulatory site, to reduce DAP's activity or affinity for substrate. In some aspects, the inhibitor may reversibly bind to DAP by non-covalent interactions, such as hydrogen bonding, ionic bonding, or hydrophobic interaction. Reversible inhibitors may act by competitive inhibition, uncompetitive inhibition, non-competitive inhibition, or mixed inhibition.

In other aspects, the inhibitor may be an irreversible inhibitor having a reactive functional group that covalently modifies DAP by reacting with amino acids of DAP to form covalent adducts. Because the irreversible inhibitor binds to the enzyme, the covalent adduct has a greater mass than unmodified DAP. The presence of an irreversible inhibitor can be identified by mass spectrometry or by using DAP-specific antibody in Western blot analysis.

A compound can be differentiated as being effective to increase DAP activity as opposed to being effective to increase DAP expression by several techniques.

In one aspect, the assay includes a cell free assay system that is not capable of gene expression. By one approach, a method is provided for identifying a compound effective to increase DAP activity in mammalian pancreatic islet cells, the method comprising: contacting DAP and Ang II with a test compound; measuring the level of Ang II; and determining if the level of Ang II is lower when treated with the test compound than an otherwise identical control containing DAP and Ang II that has not been contacted with the test compound. By one approach, the contacting step is carried out at conditions similar to physiological conditions in pancreatic islet cells. In one aspect, the contacting step is carried out in an aqueous solution at a temperature of about 32° to about 40°C, in another aspect about 35°C to about 39°C, and in another aspect at a temperature of about 37°C and at a pH of about 7.2 to about 7.6, in another aspect at about 7.4. By one approach, the aqueous solution is phosphate buffered saline (PBS). At least in some approaches, the DAP and/ or Ang II used is in a substantially purified form.

In one aspect, DAP and Ang II are contacted with the test compound for an amount of time effective for DAP to degrade at least about 10 percent of the Ang II present so that differences in activity between the test sample and the control sample can be detected. For example, the DAP and Ang II is contacted with the test compound for at least about 30 minutes, in another aspect at least about 45 minutes, and in another aspect at least about 60 minutes, or any other amount of time effective for a difference in activity to be observed between a control and test sample. By this approach, compounds that are effective cofactors or enzyme activators can be identified.

In another aspect, an islet cell lysate can be prepared and contacted with purified DAP and Ang II to determine if there is a compound present in the cell lysate that acts to decrease or inhibit DAP activity compared to an otherwise identical control that does not include cell lysate. Suitable islet cells include cells capable of producing DAP and Ang II. For example, MIN6 cells, which is a cell line derived from in vivo immortalized insulin-secreting pancreatic beta cells, can be used. The cell lysate can be prepared after culturing islet cells to suitable confluence, such as about 80 percent confluence, washing the cells with PBS, and lysing the cells with a freeze/ thaw process, such as, for example, by freezing at -20°C, -80°C, or in dry ice/ethanol bath and thawing in a room temperature or 37°C water bath. Generally, more than one freeze/thaw cycles may be necessary for efficient lysis of the cells. Other lysis techniques may be used, if desired, including homogenization, sonication, or chemical lysis, but the lysis technique selected should not adversely impact the enzymatic activity of DAP. The recovered lysate should be incapable of protein expression. Samples are then prepared containing purified DAP and Ang II which are then contacted with and without the lysate to determine if the lysate contains an inhibitor of DAP activity.

If an inhibitor is present, the sample contacted with the lysate will contain higher levels of Ang II after a period of incubation than the sample not containing the lysate. In one aspect, the period of incubation may be at least about 30 minutes, in another aspect at least about 45 minutes, and in another aspect at least about 60 minutes, or any other amount of time effective for a difference in activity to be observed between a control and test sample.

If it is found that there is an inhibitor of DAP activity in the cell lysate, a screening method is provided for identifying a compound that interferes with the DAP inhibitor and thereby increases DAP activity in the mammalian pancreatic islet cells. The method comprises contacting an islet cell lysate with a test compound and DAP and Ang II; measuring the level of Ang II; and determining if the level of Ang II is lower when treated with the test compound than an otherwise identical control (i.e., containing the cell lysate, DAP, and Ang II) that has not been contacted with the test compound. At least in some approaches, the DAP and/ or Ang II used is in a substantially purified form.

At least in some approaches, the cell lysate is treated to separate protein from smaller molecules to further characterize the inhibitor. A variety of techniques can be used. For example, a density gradient fractionalization system, which separates by size, may be used to identify and/ or isolate cell lysate components of interest. Membrane filtration may also be used, if desired, with a membrane of suitable molecular weight cutoff.

Protein levels can be measured by conventional levels. By one exemplary approach, the islet cells can be grown in DMEM, supplemented with 10 percent fetal calf serum (FCS), 10 mM glucose, and 2 mmol/l glutamine, and are grown to suitable confluence, such as at least about 80 percent confluence, prior to harvesting. The cells are generally grown at a temperature of about 35°C to about 39°C, in another aspect about 37°C. The cell lysate can be prepared after culturing islet cells to about 80 percent confluence, washing the cells, such as with PBS, and lysing the cells with a freeze/thaw process as previously described. The levels of DAP and Ang II levels can then be detected by conventional techniques, such as Western blot analysis, ELISA, or a method based thereon using an antibody that recognizes DAP and/or Ang II. Other conventional methods of detecting protein levels may be used, if desired.

Ang II levels can be detected by conventional techniques. By one approach, Ang II levels can be measured using the AssayMax Ang II ELISA Kit (Cayman Chemical, Ann Arbor, Michigan). By this approach, standards or samples can be incubated in a 96-well plate with biotinylated anti-Ang II antibody for 2 hours. After the washings, Streptavidin-Peroxidase Conjugate is added for 30 minutes. The final reaction is developed with the substrate chromogen and read at 450 nm by an ELISA reader. Ang II levels in the samples can be calculated from an Ang II standard curve run with each assay.

At least in some approaches, a test compound that increases the activity of DAP by at least about 20 percent, in another aspect at least about 30 percent, in another aspect at least about 30 percent, in another aspect at least about 40 percent, in another aspect at least about 50 percent, in another aspect at least about 60 percent, and in yet another aspect at least about 75 percent, can be selected as a compound that increases DAP activity and decreases Ang II levels in mammalian pancreatic islet cells.

### Screening Method for a Compound that Increases DAP Expression in Mammalian Pancreatic Islet Cells

By another approach, methods are provided for identifying compounds which are effective to increase the expression of DAP in mammalian pancreatic islet cells. By "increased expression" is meant that the rate of gene transcription is increased or mRNA stability is increased such that the amount of DAP or the amount of mRNA that encodes DAP produced is measurably higher when pancreatic islet cells are treated with the test compound than without the test compound. In one aspect, expression is increased by at least about 10 percent, in another aspect at least about 20 percent, in another aspect at least about 30 percent, in another aspect at least about 40 percent, and in yet another aspect at least about 50 percent.

In one aspect, the compound may effect increased expression of DAP by acting as a transcription factor. For example, the compound may promote recruitment or stabilization of RNA polymerase to the *DNPEP* gene. In another aspect, the compound may act as a transcription factor by recruiting a coactivator to the *DNPEP* gene. A transcription factor generally includes one or more DNA-domains for attachment to portions of the DNA adjacent the gene to be regulated. The transcription factor may bind to either the enhancer or promoter region of the *DNPEP* gene. In yet another aspect, the compound may act as a coactivator (a substance that works with one or more transcription factors) to increase the rate of transcription from the *DNPEP* gene.

In another aspect, the compound may effect increased expression of DAP by binding to a repressor or corepressor of gene transcription from the *DNPEP* gene. Repressors generally act by attaching to the operator of the gene and prevent RNA polymerase from attaching to the gene and transcribing the gene to produce mRNA. A corepressor acts by binding to the repressor so that the repressor binds more tightly to the operator. In one aspect, the compound may act to increase DAP expression by binding to the operator portion of the *DNPEP* gene, thereby preventing binding of a repressor. In another aspect, the compound may bind to the repressor, thereby preventing binding of the repressor to the gene. Providing such compounds to islet cells is expected to result in increased DAP expression.

In one aspect, a screening method is provided for identifying a compound effective to increase DAP expression in mammalian pancreatic islet cells, the method comprising: culturing mammalian islet cells capable of producing DAP in the presence of a test compound; measuring the level of *DNPEP* gene expression; and determining if the level of *DNPEP* gene expression is higher when treated with the test compound than an otherwise identical control not cultured with the test compound.

In another aspect, a screening method is provided for identifying a compound effective to increase DAP expression in mammalian pancreatic islet cells, the method comprising: culturing mammalian islet cells capable of producing DAP in the presence of a test compound; measuring the level of *DNPEP* gene expression; and determining if the level of *DNPEP* gene expression is higher when treated with the test compound than an otherwise identical control not cultured with the test compound. The level of gene expression can be determined by measuring the amount of DAP and/or the amount of mRNA that encodes DAP. By another approach, the method may further comprise measuring the level of Ang II and determining if the level of Ang II is lower when treated with the test compound.

In one aspect, the mammalian islet cells are contacted with the test compound for an amount of time sufficient to detect increased transcription from *DNPEP.* In another aspect, the mammalian islet cells are contacted with the test compound for an amount of time sufficient to detect increased transcription from *DNPEP* and for any DAP produced to degrade at least about 10 percent of the Ang II present so that differences in expression levels between the test sample and the control sample can be detected. For example, the cells are contacted with the test compound for at least about 30minutes and in another aspect at least about 60 minutes.

Any mammalian islet cells capable of producing DAP and Ang II can be used in the methods described herein. For example, suitable islet cells include, for example, MIN6 cells, which is a cell line derived from in vivo immortalized insulin-secreting pancreatic beta cells.

Protein levels can be measured by conventional methods. By one approach, the islet cells can be grown in DMEM, supplemented with 10% FCS, 10 mM glucose, and 2 mmol/l glutamine, and are grown to and are grown to suitable confluence, such as at least about 80 percent confluence, prior to harvesting. The cells are generally grown at a temperature of about 35°C to about 39°C, in another aspect about 37°C.. The cells can then be washed, such as with PBS, and lysed, such as by freeze/thaw as described above. The levels of DAP and Ang II levels in the lysate can then be detected by conventional techniques, such as Western blot analysis, ELISA, or a method based thereon using an antibody that recognizes DAP. Other conventional methods of detecting protein levels may be used, if desired. By one approach, Ang II levels can be detected by conventional techniques.

By one approach, Ang II levels can be measured using the AssayMax Ang II ELISA Kit (Cayman Chemical, Ann Arbor, Michigan). By this approach, standards or samples can be incubated in a 96-well plate with biotinylated anti-Ang II antibody for 2 hours. After the washings, Streptavidin-Peroxidase Conjugate is added for 30 minutes. The final reaction is developed with the substrate chromogen and read at 450 nm by an ELISA reader. Ang II levels in the samples can be calculated from an Ang II standard curve run with each assay.

The amount of mRNA can be measured according to conventional methods. The mRNA is isolated from the islet cells. For example, Northern blot analysis using as the probe a nucleic acid capable of binding to the mRNA, or Reverse Transcription PCR using as the primer a nucleic acid capable of hybridizing to the mRNA a portion thereof. Other conventional methods of detecting mRNA levels may also be used, if desired.

By one approach, small interfering RNA (siRNA) can be used to confirm that the test compound is effective for increasing DAP expression, rather than increasing DAP activity. By this approach, siRNA is provided that is sufficiently homologous to a portion of mRNA encoding DAP such that the siRNA interferes with the translation of the DAP mRNA. The siRNA can be transfected into the cells using an appropriate vector, such as pCMV-SPORT6 (Open Biosystems, Huntsville, AL) by electroporation (BioRad Laboratories, Berkeley, CA). By use of the siRNA, the endogenous DAP mRNA level and DAP protein level should be substantially reduced while Ang II levels should increase.

At least in some approaches, a test compound that increases the expression level of DAP by at least about 20 percent, in another aspect at least about 30 percent, in another aspect at least about 30 percent, in another aspect at least about 40 percent, in another aspect at least about 50 percent, in another aspect at least about 60 percent, and in another aspect at least about 75 percent, can be selected as a compound that promotes the expression of DAP or the expression of the *DNPEP* gene for the protein and decreases Ang II levels in mammalian pancreatic islet cells.

### Delivery of One or More Additional Copies of DNPEP to Increase DAP Production

By another approach, one or more additional copies of the *DNPEP* gene can be delivered to mammalian pancreatic islet cells for increasing *in vivo* expression of DAP and thereby decreasing levels of Ang II in the cells. A variety of viral-based delivery systems, including adenoviral, retroviral, adeno-associated viral, lentiviral, and herpes simplex viral systems, can be used to introduce one or more additional copies of the *DNPEP* gene in target cells.

In one aspect, a polynucleotide encoding DAP is incorporated into a viral vector. It is possible to use such a construct to perform gene therapy for diseases that arise from mutations in the *DNPEP* gene. Conventional techniques can be used to insert a polynucleotide encoding DAP (e.g., cDNA of SEQ ID NO. 8) into the viral vector. In one aspect, the polynucleotide comprises a sequence encoding a protein a having at least about 80 percent homology, in another aspect at least about 85 percent, in another aspect at least about 90 percent, and in yet another aspect at least about 95 percent homology to SEQ ID NO. 9.

In some aspects, in the construction of the vector, the polynucleotide encoding DAP may be linked to one or more regulatory regions, including promoters, enhancers, suppressors, and the like. Promoters that may be used include both constitutive and regulated (e.g., inducible) promoters. By one approach, a cell specific promoter is used. For example, a promoter may be used so that the gene is only expressed in pancreatic alpha cells. For example, a glucagon-activated promoter can be used. A suitable glucagon-activated promoter is provided at SEQ ID NO. 10.

In some aspects, the viral vectors used herein lack at least one region necessary for replication of the virus in the target cell and are replication defective.

The vector may then be administered to the subject through *ex vivo* or *in vivo* methods. By this approach, the levels of DAP in the target cells can be increased. The increased DAP levels increase the degradation of Ang II, thereby reducing Ang II levels in the cells and preventing Ang II induced pancreas islet cell dysfunction.

### Pharmaceutical Compositions

Pharmaceutical compositions which include effective amounts of the test compounds or pharmaceutically acceptable salt thereof identified herein are also provided. In some aspects, the compositions may be useful for treating or preventing type 1 diabetes.

Pharmaceutical compositions for oral administration can be formulated using pharmaceutically acceptable carriers well known in the art in dosages suitable for oral administration. Appropriate carriers can be selected to formulate the compositions in the form of, for example, tablets, pills, dragees, capsules, liquids, gels, syrups, slurries, suspensions, and the like, for ingestion by the subject. Excipients can also be included, such as carbohydrate or protein fillers, such as sugars, including lactose, sucrose, mannitol, or sorbitol; starch from corn, wheat, rice, potato, or other plants; cellulose, such as methylcellulose, hydroxypropylmethyl-cellulose, or sodium carboxymethyl-cellulose; gums including arabic and tragacanth; and proteins such as gelatin and collagen. If desired, the pharmaceutical composition may be prepared in delayed release, sustained release, or other format. The composition may also include an enteric coating, if desired.

Injectable formulations can also be prepared, such as, for example, in the form of a solution or suspension in a non-toxic, parenterally-acceptable solvent or diluent. Exemplary pharmaceutically-acceptable carriers include saline, buffered saline, isotonic saline, Ringer's solution, dextrose, water, sterile water, glycerol, ethanol, and combinations thereof.

The disclosure also provides a method of decreasing Ang II levels in a subject having Ang II induced pancreas islet cell dysfunction, the method comprising administering to the subject a therapeutically effective amount of a test compound identified according to the methods described herein, where the test compound is effective to increase the expression or activity of DAP in mammalian pancreatic islet cells. In one aspect, the subject may have or be susceptible to type 1 diabetes. In another aspect, a method is provided for the treatment or prevention of type 1 diabetes comprising administering to the subject a therapeutically effective amount of a test compound identified according to the methods herein.

As it is known that Ang II can cause insulin resistance, it is also presently believed that reducing the Ang II content of the pancreatic islet cells may be effective to at least partially and possibly significantly reducing insulin resistance and thereby controlling type II diabetes.

As used herein, therapeutically effective amount or dose means the amount of compound which ameliorates the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions that exhibit large therapeutic indices are preferred. Data obtained from cell culture assays and animal studies can be used in formulating a range of dosage for human use. The dosage may vary depending upon the dosage form employed, sensitivity of the patient, and the route of administration. The dosage suitable for a given subject can be readily determined by one of skill in the art. Generally, dosage and administration can be adjusted to provide sufficient levels of the test compound identified herein or to maintain the desired effect.

The pharmaceutical compositions provided herein may be administered to a subject by a variety of methods. In one aspect, the compositions may be applied directly to target tissues, such as the pancreas. In one aspect, local administration to the desired tissue may be done by catheter, infusion pump or stent. Additional routes of delivery include, for example, intravenous injection, intramuscular injection, subcutaneous injection, aerosol inhalation, oral (tablet or pill form), topical, systemic, ocular, intraperitoneal and/or intrathecal delivery.

In the case of delivery of recombinant viruses as discussed above, recombinant viruses are formulated and administered in the form of doses of an appropriate number of plaque forming units (pfu). The term pfu corresponds to the infective power of a suspension of virions and is determined by infecting an appropriate cell culture and measuring the number of plaques formed. The techniques for determining the pfu titre of a viral solution are well documented in the prior art and the desired dose of recombinant virus can be readily determined by one of ordinary skill in the art.

The following Examples are provided to illustrate certain embodiments but should not be construed as limiting the scope of the disclosure.

### Examples

### Example 1: Bioinformatic Analysis

The entire sequence and predicted genes of human DNPEP-containing BAC clone (GenBank acc. no. AC053503) were analyzed by BLAST searching the National Center for Biotechnology Information databases with BLAST algorithms. The adjacent genes of *DNPEP* including *PTPRN* and *RESP18* were investigated to determine whether the orthologues could be mapped to the vicinity of *DNPEP* locus in the genome of other species. Domains or motifs were predicted with SMART (a Simple Modular Architecture Research Tool, which allows the identification and annotation of genetically mobile domains and the analysis of domain architectures; *see http:*//*smart.embl-heidelberg.de*/).

The loci of conservation of syntenic genes shared between C. *elegans* and humans were determined via database searching (NCBI, Ensembl, Xenbase G-Browse, Ensembl Metazoa, and Wormbase) the genome assemblages of 12 species including seven mammals (human, *Homo sapiens*; chimpanzee, *Pan troglodytes*; monkey, *Macaca mulatta*; cow, *Bos taurus*; dog, *Canis familiaris*; rat, *Rattus norvegicus*; mouse, *Mus musculus*;), one bird (chicken, *Gallus gallus*), one amphibian (western clawed frog, *Xenopus tropicalis*), one fish (zebra fish, *Danio rerio*), two insects (fruit fly, *Drosophila melanogaster*; mosquitoes, *Anopheles gambiae*), and one worm (nematode, *Caenorhabditis elegans*).

*DNPEP* is tandemly arranged with *PTPRN* in a conserved syntenic region. The facts that *PTPRN* was clustered with *RESP18* across mammalian species, matched the sequence at the first 200 amino acids at the luminal region, as well as expressed in the same subcellular location in islet cells of the pancreas, prompted the hypothesis that the conserved syntenic region of *PTPRN* may have additional genes sharing properties including a similar expression pattern. Thus, the 163-kb PTPRN-containing BAC clone (RP11-747C8) on human chromosome 2q35 was analyzed. Orthologs of *PTPRN, RESP18* and *DNPEP* were found clustered in human genome were also present in the same order located on mouse chromosome 1. Such a syntenic segment (*PTPRN-RESP18-DNPEP* in order) was found in all seven of the mammalian species examined, including *Homo sapiens* (chromosome 2q35), *Pan troglodytes* (Chr. 2b), *Macaca mulatta* (Chr. 12), *Bos taurus* (Chr. 2), *Canis lupus familiaris* (Chr. 37), *Rattus norvegicus* (Chr. 9q33), and *Mus musculus* (Chr. 1).

To determine if the syntenic block was evolutionarily conserved in non-mammal animals, genomes in more species including *Danio rerio, Drosophila melanogaster,* and *C*. *elegans* were searched and analyzed. The ortholog of *RESP18* was not found in any non-mammal species examined. The ortholog of *DNPEP* was not found in the insect species of either *Drosophila melanogaster* or *Anopheles gambiae.* However, ortholog genes of *PTPRN* and *DNPEP* were found in all other four examined species including *Gallus gallus* (Chr. 7), *Xenopus tropicalis* (Chr. location, unknown), *Danio rerio* (Chr. 6) and *C*. *elegans* (Chr. III), suggesting that *PTPRN-DNPEP* is a several million years old syntenic segment. It also found that the cluster was duplicated in *Danio rerio* (Chr. 9 and Chr. 6). However, the *Dnpep* gene on Chr. 9 of *Danio rerio* was missing. In C. *elegans,* there are 17 other genes present in the region (less than 100 kb) between *Ptprn* (also known as *ida-1*) and *Dnpep* (e.g., F01F1.9). No identical expression patterns were identified in the 17 genes according to the Worm Database. Moreover, none of these 17 genes were syntenically mapped to the *PTPRN-DNPEP-*containing regions in other species.

### Example 2: Immunofluorescent Staining of Mouse Pancreas Sections

Paraffin embedded slides of mouse pancreas tissue were prepared and immunofluorescent staining was performed by standard methods. Microscopic analysis was completed with a Zeiss Axiophot microscope (Columbus, OH, USA) for immunofluorescence (Leica Microsystems, Wetzlar, Germany). DAP-specific rabbit polyclonal antibody was a gift from Dr. S. Wilk. Antibody to RESP18 was kindly provided by Dr. M. Schiller. Antibody to PTPRN was obtained from Santa Cruz Biotechnology (Santa Cruz, CA, USA). Anti-insulin, anti-glucagon, and anti-somatostatin mouse monoclonal antibodies were purchased from Sigma (St. Louis, MO, USA).

As shown in FIGS. 2A and 2B, PTPRN and RESP18 were both expressed in islet cells with a stronger signal in the peripheral region. As shown in FIG. 2C, results showed that DAP was also localized in peripheral cells of islets. This specific pattern suggested that DAP was either expressed in glucagon-positive alpha cells or somatostatin-positive delta cells. Further analysis confirmed that DAP fluorescent signal was completely colocalized with glucagon as detected by anti-glucagon monoclonal antibody, as shown in FIGS. 2D and 2E, but not colocalized with either insulin beta cells or somatostatin delta cells.

### Example 3: Gene-Specific Expression Analysis

The Allen Brain Atlas (ABA), a publicly accessible online gene expression digital library widely cited in numerous papers, supports gene-specific expression analysis in mouse brain by providing an automated high throughput *in situ* hybridization internet platform (*see, e.g.,* Lein et al., Nature, 445, 168-176 (2007)). *In situ* hybridization data for expression of each gene in adults were extracted from the ABA. All ABA image-based, *in situ* hybridization data are spatially registered to the common anatomic framework of the Allen Reference Atlas (ARA) (*see* Dong et al., The Allen Reference Atlas:A Digital Color Brain Atlas of the C57BL/6J Male Mouse (Wiley, Hoboken, NJ, 2008), pp. 1-376) with a standard coordinate system and hierarchical ontology. The Anatomic Gene Expression Atlas (AGEA) in principle enables users to view a comprehensive list of genes expressed in any given three-dimensional voxel of the mouse brain atlas by using the Gene Finder application (Ng et al., Nat. Neurosci., 12, 356-362 (2009)).

Expression levels in different brain regions were downloaded as xml files and processed with Microsoft Excel 2007. Sagittal planes of the spinal cord were taken at cervical spinal level (C1) for further analysis of *DNPEP, RESP18,* and *PTPRN* expression data. To examine whether *DNPEP* expression in neuronal tissues is similar to *PTPRN* and *RESP18,* the area where messenger RNAs that encode DAP, RESP18 and PTPRN proteins are expressed in adult (56 days) mouse brain tissues, using the ABA data sets was analyzed. The probes used for *in situ* hybridization were 781-bp of mouse *DNPEP* (nt 1058-1819, GenBank acc. no., NM_001110831), 591-bp of mouse *RESP18* (nt 60-650, GenBank acc. no., NM_009049), and 713-bp of mouse *PTPRN* (nt 1947-2659, GenBank acc. no., NM_008985), respectively. The identities/ similarities among these probes were low (<40-45%) with no significance. However, the expression patterns of *Dnpep, Resp18* and *Ptprn* were very similar, present in various regions of brain as shown in FIG. 3. Detailed analysis showed that higher expression levels of *Dnpep, Resp18* and *Ptprn* were in the midbrain, pons, medulla, thalamus, main olfactory bulb, and hippocampus regions. Lower expression levels of these genes were found in the subthalamic nucleus, hippocampal region, cerebral cortex, caudate putamen, and cerebellum as indicated respectively in FIG. 3.

*Dnpep, Resp18* and *Ptprn* were expressed with similar patterns in all twenty segments of the spinal cord from the cervical to lumber spinal cord. FIG. 4 illustrates that the mRNAs of the three genes were similarly distributed in the uppermost cervical segment of the spinal cord of adult mice, mainly located in neuron-rich regions of gray matter.

### Example 4: Immunoelectron microscopy: DAP was found in secretory granules.

To further examine subcellular localization of DAP in islet cells, immunoelectron microscopy analysis with anti-DAP antibody was conducted. Mouse pancreatic islets were isolated, fixed, and embedded by standard methods. *See, e.g.,* Zhang et al., J. Endocrinol., 195, 313-321 (2007). Immunogold Electron Microscopy was performed as follows. Briefly, the sections were exposed to blocking solution containing 2% BSA, 2% normal goat serum, and 0.1% fish gelatin in PBS containing 0.05% Tween-20 (PBST) for 60 min and incubated with anti-DAP polyclonal rabbit antibody (1/10 dilution) and anti-glucagon monoclonal mouse antibody (1/50 dilution). The primary antibodies were detected with 5 nm diameter colloidal gold particles conjugated to goat-anti-rabbit IgG and 10 nm diameter colloidal gold particles conjugated to goat-anti-mouse IgG respectively (Ted Pella, Redding, CA, USA).

Electron micrographs showed that DAP was associated with lysosomal-like structures (FIG. 5A) as well as secretory granules in islet alpha cells (FIG. 5B). Quantitative analysis of DAP particles per square micrometer in 20 electron microscopic images revealed that there were 84 ± 13 particles around lysosome and 32 ± 9 in secretory granules comparing to 9 ± 3 particles in the cytoplasm (mean ± S.D.; P< 0.01). By double immunolabeling with rabbit antibody to DAP (5 nm colloidal gold particles conjugated to goat anti-rabbit antibody) and mouse antibody to glucagon (10 nm of colloidal gold conjugated with goat anti-mouse antibody), we found that DAP was colocalized with glucagon in islet alpha cells (FIG. 5C).

### Example 5: DAP Enzymatic Activity

To examine whether the DAP aminopeptidase activity in cells was reconciled with the subcellular distribution shown in the electron microscopic analysis, DAP enzymatic activities in fractionated mouse brain tissues was measured. Since DAP was found in secretory granules in islet cells, it was assumed that DAP could be colocalized with vesicles in neuronal cells of brain. Synaptosomes that contain secretory vesicles and mitochondria of presynaptic terminal and postsynaptic membranes were prepared from brain tissues by classical subcellular fractionation techniques. Lysosome-, microsome-, and nuclei-enriched fractions were also prepared for enzymatic activity analysis. Synaptosome that contains vesicles was enriched as previously described. *See, e.g.,* Bai et al., Subcell. Biochem., 43, 77-98 (2007).

Briefly, whole mouse brain was homogenized in buffer (0.32 M sucrose, 20 mM HEPES, pH 7.4, with protease inhibitor cocktail, Sigma) and centrifuged at 1000 g for 10 minutes to pellet the membrane fragments and nuclei. The supernatant was then centrifuged at 17,000 g for 15 minutes to obtain the pellet-containing synaptosomes contaminated with mitochondria and microsomes. This crude synaptosome fraction was further purified by using a discontinuous sucrose density gradient consisting of a 0.8 M sucrose layer on the top and a 1.2 M sucrose layer on the bottom. The pure synaptosomal fraction can be obtained from the interface of 0.8 M and 1.2 M sucrose after 90 minutes centrifugation at 54,000 g. The lysosome- or nuclei-containing fraction was then enriched with other appropriate reagents (Instructions 89839 and 89841 respectively, Pierce, Rockford, IL, USA).

Membrane-bound DAP activity was measured as previously described in the buffer of 50 Tris-HCl (pH 7.4), 1 mM MnCl₂ and 0.125 mM aspartyl-beta-NA. *See, e.g.,* W. Wang et al., Mol. Biol. Evol., 24, 784-791 (2007). Relative fluorescence was converted into pmoles of product using a standard curve, constructed with increasing concentrations of beta-naphthylamine. The results were recorded as units of aminopeptidase per mg protein of analyzed tissue. One unit of aminopeptidase activity was the amount of enzyme that hydrolyzed 1 pmol of aminoacyl-beta-naphthylamide per minute. Protein concentrations were measured by Micro BCA Protein Assay (Pierce). Data was evaluated using one-way analysis of the variance (ANOVA test), followed by the DMS test for comparisons between more than two groups.

Data showed that the highest activities of DAP (∼400 units) were detected in the synaptosomal and the lysosomal fractions as shown in FIG. 6. Although the electron microscopic analysis did not show the presence of DAP in ER and Golgi or nuclei, lower activities were also found in the fractions of microsome (∼142 units) and nuclei (∼57 units) as shown in FIG. 6. It cannot be excluded, however, that the lower activities were attributed by small amount heterogenous fractions of cytosol proteins that contained the DAP protein.

### Example 6: Modulation of Angiotensin II Levels by DAP

DAP proteolytic activities are responsible for deleting the N-terminal aspartic residues (Asp) of both Ang I (1-14) (Asp-Arg-Val-Tyr-Ile-His-Pro-Phe-His-Leu-Leu-Val-Tyr-Ser) and Ang II (1-8) (Asp-Arg-Val-Tyr-Ile-His-Pro-Phe), which lead to the production of Ang I* (des-Asp-Ang I) and Ang III (des-Asp-Ang II), respectively. Since the angiotensin-renin system (ARS) is abundantly expressed in local islet a cells and MIN6 cells, whether DAP expression modulates the Ang II levels in islet cells was investigated.

The DAP gene was either knocked down with DAP RNAi construct or overexpressed by transient transfection of DAP-pCMV in MIN6 cells.

The full-length DAP construct or the control vector was transfected into MIN6 cells. After culturing for 48 hours, MIN6 cells and the culture media were harvested for measurement of DAP and Ang II levels. Higher expression level of DAP (2.1-fold ± 0.6) in DAP-pCMV-transfected MIN6 cells compared to that in the vector-transfected cells was detected by Western blots. Ang II levels in MIN6 cell lysates and the cultured media were measured after the transfection and compared to the control transfected cells. Ang II-specific ELISA kit (Cayman Chemical) was used for the measurements.

In the DAP-transfected cells, intracellular Ang II levels were 28 percent lower than that in vector-treated cells (22 ± 4 pg/mg vs. 34 ± 3 pg/mg protein respectively in mean ± SEM, p < 0.01) and the extracellular Ang II levels were 35 percent lower than that in control cells (22 ± 3 pg/mg vs. 34 ± 4 pg/mg protein respectively, p < 0.01). The data represented as percentages of controls are from at least three independent experiments. The decrease of both intracellular and extracellular Ang II levels in DAP transfected MIN6 cells, as shown in FIG. 7, suggests an enhanced hydrolysis of Ang II.

It was also investigated whether endogenous DAP levels affect the Ang II levels. Dnpep siRNA and control siRNA were purchased (Qiagen, Germantown, MD). The target sequences were nucleotides 328-348 (SEQ ID NO. 11: AGAGGCCATCCAGGCCACATT) of mouse Dnpep cDNA (GenBank NM_001110831; SEQ ID NO. 12). MIN6 cells were maintained in DMEM, supplemented with 10% FCS, 10 mM glucose, 2 mmol/l glutamine, 100 U/ml penicillin, and 0.1 mg/ml streptomycin.

The cells were transfected with 2 µg of Dnpep siRNA of SEQ ID NO. 13, non-silencing control siRNA, or a full-length of Dnpep cDNA (SEQ ID NO. 12) in pCMV-SPORT6 (Open Biosystems, Huntsville, AL) by electroporation (BioRad Laboratories, Berkeley, CA). A total of 1×10⁵ of the cells was seeded into 6-well culture plates coated with poly-L-lysine and cultured for 24 h. The transfection efficiency was approximately 50 percent according to a co-transfected GFP marker.

After 48 hours culture, cells and culture media were collected. Real-time PCR (RT-PCR) was conducted with the primers (Assay ID Details: Mm00497488_m1*) and the 7900HT Fast Real-Time PCR System (Applied Biosystems, Foster City, CA). Western blots were performed using the ECL Reagents (GE, Piscataway, NJ).

In the DAP RNAi-transfected MIN6 cells, the endogenous DAP mRNA level was substantially knocked down by 78 percent as shown by RT-PCR analysis, and the DAP protein level decreased 70 percent compared to that in control cells by Western blots. Consequently, the intracellular Ang II levels increased up to 74 percent compared to the control cells (50 ± 6 pg/mg vs. 28.7 ± 4 pg/mg protein respectively, p < 0.01) and extracellular Ang II levels increased 57 percent compared to the control cells (52 ± 5.3 pg/mg vs. 33.1 ± 4.2 pg/mg protein respectively, p < 0.01) (FIG. 7). The data represented as percentages of controls are from at least three independent experiments. The results suggest that DAP is a major hydrolase for Ang II conversion in islet cells.

Ang II levels were measured using the AssayMax Ang II ELISA Kit (Cayman Chemical, Ann Arbor, Michigan). Briefly, standards or samples were incubated in a 96-well plate with biotinylated anti-Ang II antibody for 2 h. After the washings, Streptavidin-Peroxidase Conjugate was added for 30 min. The final reaction was developed with the substrate chromogen and read at 450 nm by an ELISA reader. Ang II levels in the samples were calculated from an Ang II standard curve run with each assay. Values (mean ±SEM) are from at least three independent experiments. ANOVA with Tukey's post hoc test was used for statistical analysis.

While the invention has been particularly described with specific reference to particular processes and embodiments, it will be appreciated that various alterations, modifications, and adaptations may be based on the present disclosure, and are intended to be within the scope of the invention as defined by the following claims.

## Claims

1. An in-vitro method for identifying a compound effective to increase aspartyl aminopeptidase (DAP) activity in mammalian pancreatic islet cells, the method comprising :
- contacting an islet cell lysate with DAP, angiotensin II and a test compound;
- measuring the level of angiotensin II; and
- determining if the level of angiotensin II is lower when treated with the test compound than an otherwise identical control method containing islet cell lysate, DAP and angiotensin II that has not been contacted with the test compound.

2. The method according to claim 1, wherein the contacting step is carried out at a temperature of 35° to 39 °C in an aqueous solution, said aqueous solution preferably having a pH of 7.2 to 7.6.

3. The method according to claim 1, wherein the method further comprises measuring activity of DAP and determining if the activity of DAP is higher when treated with the test compound than an otherwise identical control containing DAP and angiotensin II that has not been contacted with the test compound.

4. The method according to claim 1, wherein the DAP and angiotensin II are contacted with the test compound for an amount of time effective for the DAP to degrade at least about 10 percent of the angiotensin II.

5. The method according to claim 1, wherein the DAP and angiotensin II are each in substantially purified form.

6. A method for identifying a compound effective to increase DAP expression in mammalian pancreatic islet cells, the method comprising:
- culturing mammalian islet cells capable of producing DAP in the presence of a test compound;
- measuring the level of expression from the gene encoding DAP; and
- determining if the level of expression is higher when treated with the test compound than an otherwise identical control method with cells not cultured with the test compound.

7. The method according to claim 6, wherein the method further comprises measuring an amount of angiotensin II and determining if the level of angiotensin II is lower when treated with the test compound than an otherwise identical control not cultured with the test compound.

8. The method according to claim 6, wherein measuring the level of expression comprises measuring the amount of DAP in the cultured cells.

9. The method according to claim 6, wherein measuring the level of expression comprises measuring the amount of mRNA encoding DAP in the cultured cells.

10. An in-vitro method for increasing DAP expression and decreasing Ang II levels, the method comprising delivering one or more copies of a polynucleotide encoding DAP to pancreatic islet cells.

11. The method according to claim 10, wherein the one or more copies of a polynucleotide encoding DAP are delivered to the subject's pancreatic islet cells via a viral vector.

12. The method according to claim 11, wherein the viral vector includes a promoter activated in pancreatic alpha cells.

13. The method according to claim 12, wherein the promoter is a glucagon- activated promoter.

14. The method according to claim 10, wherein the polynucleotide encoding DAP comprises a sequence encoding a protein a having at least 80 percent identity to SEQ ID NO. 9.

15. The method according to claim 10, wherein the polynucleotide encoding DAP comprises a sequence encoding a protein a having at least 85 percent identity to SEQ ID NO. 9.

## Patentansprüche

1. In-vitro-Verfahren zum Identifizieren einer Verbindung, die dahingehend wirksam ist, dass sie die Aspartylaminopeptidase(DAP)-Aktivität in Pankreas-Inselzellen von Säugern erhöht, wobei das Verfahren Folgendes umfasst:
- Inkontaktbringen eines Inselzelllysats mit DAP, Angiotensin II und einer Testverbindung;
- Messen des Angiotensin-II-Spiegels; und
- Bestimmen, ob der Angiotensin-II-Spiegel bei Behandlung mit der Testverbindung niedriger ist als ein ansonsten identisches Kontrollverfahren, das Inselzelllysat, DAP und Angiotensin II enthält, das nicht mit der Testverbindung in Kontakt gebracht worden ist.

2. Verfahren nach Anspruch 1, wobei der Schritt des Inkontaktbringens in einer wässrigen Lösung bei einer Temperatur von 35° bis 39° C erfolgt, wobei die wässrige Lösung vorzugsweise einen pH-Wert von 7,2 bis 7,6 aufweist.

3. Verfahren nach Anspruch 1, wobei das Verfahren weiterhin Folgendes umfasst: Messen der DAP-Aktivität und Bestimmen, ob die DAP-Aktivität bei Behandlung mit der Testverbindung höher ist als eine ansonsten identische Kontrolle, die DAP und Angiotensin II enthält und die nicht mit der Testverbindung in Kontakt gebracht worden ist.

4. Verfahren nach Anspruch 1, wobei das DAP und Angiotensin II mit der Testverbindung so lange in Kontakt gebracht werden, daß das DAP mindestens um 10% des Angiotensin II abgebaut wird.

5. Verfahren nach Anspruch 1, wobei das DAP und Angiotensin II jeweils in im Wesentlichen aufgereinigter Form vorliegen.

6. Verfahren zum Identifizieren einer Verbindung, die dahingehend wirksam ist, dass sie die DAP-Expression in Pankreas-Inselzellen von Säugern erhöht, wobei das Verfahren Folgendes umfasst:
- Kultivieren von Säuger-Inselzellen, die fähig sind, DAP zu produzieren, in Gegenwart einer Testverbindung;
- Messen des Expressionsniveaus des für DAP codierenden Gens; und
- Bestimmen, ob das Expressionsniveau bei Behandlung mit der Testverbindung höher ist als ein ansonsten identisches Kontrollverfahren, bei dem die Zellen nicht mit der Testverbindung kultiviert werden.

7. Verfahren nach Anspruch 6, wobei das Verfahren weiterhin Folgendes umfasst: Messen einer Angiotensin-II-Menge und Bestimmen, ob der Angiotensin-II-Spiegel bei Behandlung mit der Testverbindung niedriger ist als eine ansonsten identische Kontrolle, die nicht mit der Testverbindung kultiviert wird.

8. Verfahren nach Anspruch 6, wobei das Messen des Expressionsniveaus das Messen der DAP-Menge in den kultivierten Zellen umfasst.

9. Verfahren nach Anspruch 6, wobei das Messen des Expressionsniveaus das Messen der Menge an für DAP codierender mRNA in den kultivierten Zellen umfasst.

10. In-vitro-Verfahren zum Erhöhen der DAP-Expression und Vermindern der Ang-II-Spiegel, wobei das Verfahren Folgendes umfasst: Abgeben von einer oder mehreren Kopien eines für DAP codierenden Polynukleotids an Pankreas-Inselzellen.

11. Verfahren nach Anspruch 10, wobei die eine oder mehreren Kopien eines für DAP codierenden Polynukleotids über einen Virusvektor an die Pankreas-Inselzellen des Individuums abgegeben werden.

12. Verfahren nach Anspruch 11, wobei der Virusvektor einen in Pankreas-alpha-Zellen aktivierten Promoter beinhaltet.

13. Verfahren nach Anspruch 12, wobei es sich bei dem Promoter um einen von Glucagon aktivierten Promoter handelt.

14. Verfahren nach Anspruch 10, wobei das für DAP codierende Polynukleotid eine Sequenz umfasst, die für ein Protein mit mindestens 80 Prozent Identität zu SEQ ID NO: 9 codiert.

15. Verfahren nach Anspruch 10, wobei das für DAP codierende Polynukleotid eine Sequenz umfasst, die für ein Protein mit mindestens 85 Prozent Identität zu SEQ ID NO: 9 codiert.

## Revendications

1. Procédé in vitro d'identification d'un composé efficace pour augmenter l'activité aspartyl-aminopeptidase (DAP) dans des cellules d'îlot pancréatique de mammifère, le procédé comprenant :
- la mise en contact d'un lysat de cellules d'îlot avec DAP, l'angiotensine II et un composé d'essai ;
- la mesure du taux d'angiotensine II ; et
- la détermination du fait que le taux d'angiotensine II est ou non plus faible lors du traitement avec le composé d'essai par rapport à un procédé témoin identique par ailleurs contenant un lysat de cellules d'îlot, DAP et l'angiotensine II qui n'a pas été mis en contact avec le composé d'essai.

2. Procédé selon la revendication 1, dans lequel l'étape de mise en contact est conduite à une température de 35 à 39 °C dans une solution aqueuse, ladite solution aqueuse ayant de préférence un pH de 7,2 à 7,6.

3. Procédé selon la revendication 1, le procédé comprenant en outre la mesure de l'activité de DAP et la détermination du fait que l'activité de DAP est ou non plus élevée lors du traitement avec le composé d'essai par rapport à un témoin identique par ailleurs contenant DAP et l'angiotensine II qui n'a pas été mis en contact avec le composé d'essai.

4. Procédé selon la revendication 1, dans lequel le DAP et l'angiotensine II sont mis en contact avec le composé d'essai pendant une durée efficace pour que DAP dégrade au moins environ 10 pour cent de l'angiotensine II.

5. Procédé selon la revendication 1, dans lequel le DAP et l'angiotensine II sont chacun sous forme sensiblement purifiée.

6. Procédé d'identification d'un composé efficace pour augmenter l'expression de DAP dans des cellules d'îlot pancréatique de mammifère, le procédé comprenant :
- la culture de cellules d'îlot de mammifère capables de produire DAP en présence d'un composé d'essai ;
- la mesure du taux d'expression à partir du gène codant pour DAP ; et
- la détermination du fait que le taux d'expression est ou non plus élevé lors du traitement avec le composé d'essai par rapport à un procédé témoin identique par ailleurs avec des cellules non cultivées avec le composé d'essai.

7. Procédé selon la revendication 6, le procédé comprenant en outre la mesure d'une quantité d'angiotensine II et la détermination du fait que le taux d'angiotensine II est plus faible lors du traitement avec le composé d'essai par rapport à un témoin identique par ailleurs non cultivé avec le composé d'essai.

8. Procédé selon la revendication 6, dans lequel la mesure du taux d'expression comprend la mesure de la quantité de DAP dans les cellules cultivées.

9. Procédé selon la revendication 6, dans lequel la mesure du taux d'expression comprend la mesure de la quantité d'ARNm codant pour DAP dans les cellules cultivées.

10. Procédé in vitro d'augmentation de l'expression de DAP et de diminution des taux d'Ang II, le procédé comprenant le transfert d'une ou plusieurs copies d'un polynucléotide codant pour DAP dans des cellules d'îlot pancréatique.

11. Procédé selon la revendication 10, dans lequel les une ou plusieurs copies d'un polynucléotide codant pour DAP sont transférées dans les cellules d'îlot pancréatique du sujet par l'intermédiaire d'un vecteur viral.

12. Procédé selon la revendication 11, dans lequel le vecteur viral comprend un promoteur activé dans des cellules alpha pancréatiques.

13. Procédé selon la revendication 12, dans lequel le promoteur est un promoteur activé par le glucagon.

14. Procédé selon la revendication 10, dans lequel le polynucléotide codant pour DAP comprend une séquence codant pour une protéine ayant au moins 80 pour cent d'identité avec SEQ ID NO. 9.

15. Procédé selon la revendication 10, dans lequel le polynucléotide codant pour DAP comprend une séquence codant pour une protéine ayant au moins 85 pour cent d'identité avec SEQ ID NO. 9.
